# EUROPEAN PATENT SPECIFICATION

(11) **EP 2 172 481 B1**
(45) Date of publication and mention of the grant of the patent: **29.10.2014**
(21) Application number: 08075893.1
(22) Date of filing: 20.11.2008
(51) Int. Cl.: C07K 16/00, C07K 16/20, C07K 16/46

(54) **Proteolytically stable antibody formats**
Proteolytisch stabile Antikörperformate
Formats d'anticorps stables de manière protéolytique

(30) Priority: 06.10.2008 DE 102008050749; 07.10.2008 DE 102008050757
(43) Date of publication of application: 07.04.2010
(73) Proprietor: Novoplant GmbH, 06466 Gatersleben (DE)
(72) Inventor: Giersberg, Martin, 06484 Quedlinburg (DE); Conrad, Udo, 06458 Hausneindorf (DE); Kiprijanov, Sergej, 0768 Oslo (NO); Hähnel, Sigrun, 06484 Quedlingburg (DE); Doreen Jahn, 16341 Panketal (DE)
(74) Representative: Boeckh, Tobias

(56) References cited:
- WO-A-00/61618
- WO-A-03/087324
- WO-A-2005/082004
- WO-A1-01/57226
- WO-A1-02/02781
- WO-A1-99/32645
- WO-A1-99/55369
- WU A M ET AL: "Multimerization of a chimeric anti-CD20 single-chain Fv-Fc fusion protein is mediated through variable domain exchange" PROTEIN ENGINEERING, OXFORD UNIVERSITY PRESS, SURREY, GB, vol. 14, no. 12, 1 January 2001 (2001-01-01), pages 1025-1033, XP002982160 ISSN: 0269-2139
- RAATS J M H ET AL: "Recombinant antibody expression vectors enabling double and triple immunostaining of tissue culture cells using monoclonal antibodies" EUROPEAN JOURNAL OF CELL BIOLOGY, WISSENSCHAFLICHE VERLAGSGESELLSCHAFT, STUTTGART, DE, vol. 84, no. 4, 22 April 2005 (2005-04-22), pages 517-521, XP025312756 ISSN: 0171-9335 [retrieved on 2005-04-22]
- ILL C ET AL: "Design and construction of a hybrid immunoglobulin domain with properties of both heavy and light chain variable regions" PROTEIN ENGINEERING, OXFORD UNIVERSITY PRESS, SURREY, GB, vol. 10, no. 8, 1 August 1997 (1997-08-01), pages 949-957, XP002110047 ISSN: 0269-2139
- LU DAN ET AL: "Di-diabody: A novel tetravalent bispecific antibody molecule by design" JOURNAL OF IMMUNOLOGICAL METHODS,, vol. 279, no. 1-2, 1 August 2003 (2003-08-01), pages 219-232, XP002542445
- HAYDEN M S ET AL: "SINGLE-CHAIN MONO- AND BISPECIFIC ANTIBODY DERIVATIVES WITH NOVEL BIOLOGICAL PROPERTIES AND ANTITUMOUR ACTIVITY FROM A COS CELL TRANSIENT EXPRESSION SYSTEM" THERAPEUTIC IMMUNOLOGY, BLACKWELL SCIENTIFIC PUBL. LONDON, GB, vol. 1, no. 1, 1 January 1994 (1994-01-01), pages 3-15, XP009023719 ISSN: 0967-0149
- WALLACH M ET AL: "Passive immunization of chickens against Eimeria maxima infection with a monoclonal antibody developed against a gametocyte antigen." INFECTION AND IMMUNITY FEB 1990, vol. 58, no. 2, February 1990 (1990-02), pages 557-562, XP002543155 ISSN: 0019-9567
- MULLER K M ET AL: "The first constant domain (CH1 and CL) of an antibody used as heterodimerization domain for bispecific miniantibodies", FEBS LETTERS, ELSEVIER, AMSTERDAM, NL, vol. 422, no. 2, 30 January 1998 (1998-01-30), pages 259-264, XP004261818, ISSN: 0014-5793, DOI: DOI:10.1016/S0014-5793(98)00021-0
- STRACHAN G ET AL: "Binding characteristics of anti-atrazine monoclonal antibodies and their fragments synthesised in bacteria and plants", BIOSENSORS AND BIOELECTRONICS, ELSEVIER BV, NL, vol. 13, no. 6, 1 September 1998 (1998-09-01), pages 665-673, XP026050118, ISSN: 0956-5663, DOI: DOI:10.1016/S0956-5663(98)00022-0 [retrieved on 1998-09-01]
- 'AJ307476.1 : Mus musculus partial 17.2.25-D-Jh4 gene for immunoglobulin heavy chain, sequence t14b', [Online] 16 February 2001, European Nucleotide Archive (ENA)/EMBL-Bank Retrieved from the Internet: <URL:http://www.ebi.ac.uk/ena/data/view/AJ3 07476.1> [retrieved on 2011-07-13]
- 'Z37146.1 : M.musculus rearranged anti-MPO antibody immunoglobulin kappa chain variable region', [Online] 14 September 1994, European Nucleotide Archive (ENA)/EMBL-Bank Retrieved from the Internet: <URL:http://www.ebi.ac.uk/ena/data/view/Z37 146.1> [retrieved on 2011-07-13]
- WILKIE D C ET AL: "The effect of hen-egg antibodies on Clostridium perfringens colonization in the gastrointestinal tract of broiler chickens", PREVENTIVE VETERINARY MEDICINE, ELSEVIER, AMSTERDAM, NL, vol. 74, no. 4, 16 June 2006 (2006-06-16), pages 279-292, XP027898802, ISSN: 0167-5877 [retrieved on 2006-06-16]

## Description

### Summary

The present invention relates to a new antigen-binding polypeptide and/or an antibody construct. Furthermore the invention relates to a polynucleotide encoding said polypeptide, a vector comprising the polynucleotide and a prokaryotic or eukaryotic host cells comprising the polynucleotide, the vector and/or the polypeptide. The invention also relates to plants, parts thereof or microorganisms containing the polynucleotide, the vector and/or the polypeptide. Furthermore the invention relates to a composition comprising the polynucleotide, the polypeptide and/or the antibody construct and an acceptable excipient, carrier, buffer and/or stabilizer.

### Background

The immune system of vertebrates is a complex defence system that is responsible for distinguishing self from everything foreign to the organism, and for protecting the organism against infections and foreign substances. The immune system is divided into innate immune system, and acquired or adaptive immune system, each of which contains humoral and cellular components.

Humoral immunity refers to antibody production, and the accessory processes that accompany it. It also refers to the effector functions of antibodies, for example pathogen and/or toxin neutralization, classical complement activation, opsonin promotion of phagocytosis and pathogen elimination.

Cell-mediated immunity involves the activation of macrophages, natural killer cells, antigenspecific cytotoxic T-lymphocytes and the release of various cytokines in response to an antigen.

Despite all defence mechanisms, pathogens have evolved elaborate strategies to evade the immune response of an organism.

Therefore the development of new antigen-binding fragments is a constant need.

Antibodies, also known as immunoglobulins, are an indispensable component of the defence mechanism against potential pathogens. Antibodies are globular plasma proteins with a molecular weight of approximately 150 kDa. A full-length antibody as it exists naturally is an immunoglobulin molecule comprised of four peptide chains, two heavy (H) chains (about 50-70 kDa when full length) and two light (L) chains (about 25 kDa when full length) interconnected by disulfide bonds. The amino terminal portion of each chain includes a variable region primarily responsible for antigen recognition. The carboxy-terminal portion of each chain defines a constant region primarily responsible for effector function.

Light chains are classified as kappa or lambda and characterized by a particular constant region. Each light chain is comprised of an N-terminal light chain variable region (LCVR) and a light chain constant region comprised of one domain.

Heavy chains are classified as gamma, mu, alpha, delta, or epsilon, and define the antibody's isotype as IgG, IgM, IgA, IgD, and IgE, respectively and several of these may be further divided into subclasses (isotypes) e.g., IgG1, IgG2, IgG3, IgG4, IgA1 and IgA2. Each heavy chain type is characterized by a particular constant region. Each heavy chain is comprised of an N-terminal heavy chain variable region (HCVR) and a heavy chain constant region.

The HCVR and LCVR regions can be further subdivided into regions of hypervariability, termed complementarity determining regions ("CDRs"), interspersed with regions that are more conserved, termed framework regions. The CDRs contain most of the residues which interact specifically with the antigen by forming a surface complementary to the antigen.

An antibody "Fab" fragment (fragment antigen binding) is composed of a complete light chain linked to the N-terminal part of a heavy chain, consisting of the variable region (V_{H}) and one constant domain (C_{H}1). The Fab fragment may be generated e.g. by papain hydrolysis of an antibody. The other part of an antibody is called "Fc" region (fragment crystallizable region) or "Fc" fragment. The Fc region is the tail region of an antibody that interacts with cell surface receptors called Fc receptors and some proteins of the complement system.

One of the smallest molecules with a whole antigen binding site is the so called single chain variable fragment (scFv). ScFv is a fusion of the variable regions of the heavy and light chains of immunoglobulins, linked together with a linker.

Gene technology methods such as phage display allow the production of a recombinant single-chain variable fragment (scFV) which is a truncated Fab fragment consisting of the variable region of a heavy chain (V_{H}) and the variable region of a light chain (V_{L}), which are linked to each other by a synthetic peptide.

Phage display is a standard technique for the presentation of distinct peptides, polypeptides or proteins on the surface of bacteriophages. It allows the identification of the exposed peptides, polypeptides or proteins via their binding properties. The technique is used for the establishment of libraries of peptide, polypeptide or protein variants such as different antibody fragments. For this, mammals such as mice are immunized against the antigen of interest and lymphocytes are isolated, e.g. from the spleen. Then, mRNA is isolated, and a cDNA library is generated by cloning the cDNA into a phage display vector. The phage display library generated in this way contains recombinant scFv antibody fragments. The random library is screened for protein-protein interactions, e.g. for their antigen binding specificities. Interacting clones of the library are enriched, and the phagemid DNA from individual clones can be isolated and sequenced.

Using recombinant antibody has significant advantages compared with the conventional antibody and therefore its use becoming more popular nowadays. No animals are needed in the manufacturing procedure of the recombinant antibodies; in addition, the manufacturing time is relatively short compared with the conventional method. Moreover, the quality of the final product is higher that these of the non recombinant method.

The construction of recombinant antibody fragments is based on methods of genetical engineering and the production can be carried out *in vitro.*

Antibodies play an important role in clinical applications as well as diagnostic assays. Administration of antibodies is an attractive approach to establish protective immunity, employed in passive vaccination or in therapy. They are also used as vehicles that direct a linked moiety, e.g., an active agent or a toxin, to specific sites in the organism, where the antigens recognized by the antibodies expressed. Labelled antibodies can also be used for *in vivo* or *in vitro* diagnostics.

There are two main ways to use specific antibodies to defend an organism against certain pathogens: active and passive immunisation.

Active immunization entails the introduction of an antigen into the body, which causes the body itself to generate immunity against the target. Active immunization is normally used for prophylaxis, like vaccination, but not for treatment of infections.

Passive immunization is where pre-made elements, like antibodies, of the immune system are transferred to an organism. This method of immunization begins to work very quickly, but it is short lasting, because the antibodies are naturally broken down, and if there are no B cells to produce more antibodies, they will disappear.

Passive immunization can be used if there has been a recent outbreak of a particular disease or as an emergency treatment to poisons (for example, for tetanus).

One of the drawbacks of passive immunization is the poor stability of most antibodies. Especially oral administration is not very effective, because the antibodies are denaturized and degraded in the stomach due to enzymes and the acid environment. However, oral administration is the cheapest and most convenient way, particularly for the immunization of production animals.

It is an object of the present invention to provide novel antigen-binding polypeptides, particularly antibody fragments. These polypeptides preferably neutralize invasion, have a protective and/or curative effect and are superior to the antigen-binding polypeptides known in the state of art. Especially the object of the present invention is to provide antibody constructs that can be used in passive immunization without the limitations known in the state of art.

A further object of the present invention is to provide polynucleotides and/or vectors encoding these polypeptides, prokaryotic or eukaryotic host cells comprising these polynucleotides, vectors and/or polypeptides transgenic plants, parts thereof or microorganisms containing the polynucleotides, vectors and/or polypeptides of the present invention, as well as compositions comprising said antigen-binding polypeptides.

These and further objectives are achieved by the subject-matter of the patent claims.

WO 02/02781 discloses the manufacture of a heterodimeric fusion protein, in which a bispecific diabody is stabilised by fusing the two monomeric diabody-subunits to a CH1 constant domain and a CL constant domain, respectively. The document also discloses that the diabody-CL monomer was expressed as dimeric diabody-CL fusion protein, but the functionality thereof was not tested.

Raats et al. (European Journal of Cell Biology, vol. 84, no. 4, 2005, pages 517-421) report the manufacture of a dimeric, bivalent antibody construct in which two scFvs are fused to and dimerised via two CL constant domains, respectively.

### Description

In a first aspect, the invention relates to an antibody construct, comprising two polypeptide monomers, each Polypeptide monomer comprising
- one constant light domain, wherein
   the constant domains of both monomers are operatively connected, preferred via a disulfide bond.
- one variable light domain, wherein
   the variable light domain is associated with the constant domain,
- one variable heavy domain, wherein
   the variable heavy domain is connected to the variable light domain via a linker, wherein
   said linker consists of 0-12 amino acids, whereby
   the variable light domain of each monomer is operatively associated with the variable heavy domain of the other monomer, and
   the variable light domain of each monomer forms an antigen binding site with the variable
   heavy domain of the other monomer.

The present invention relates to a dimer of a polypeptide comprising a variable domain of an immunoglobulin heavy chain (V_{H}) linked to an antibody light chain by a peptide linker, wherein the V_{H} domain of the first monomer associates with the variable domain of the light chain (V_{L}) of the second monomer to form a first antigen binding site; and the V_{H} domain of the second monomer associates with the V_{L} domain of the first monomer to form a second antigen binding site; the polypeptide linker connecting V_{H} and the light chain being of a length such that the adjacent V_{H} and V_{L} domains of the same monomer are incapable of associating with each other to form an antigen binding site.

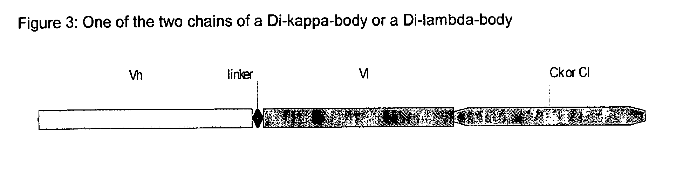

That means, in a preferred embodiment the invention provides a dimer of a polypeptide, wherein each monomer comprises three domains and a linker (Figure 3). The three domains are a constant domain of a light chain, which can be either a kappa domain or a lambda domain, a variable domain of a light chain and a variable domain of a heavy chain. The constant domain of a light chain is connected to the variable domain of a light chain. And the variable domain of a light chain is connected to the variable domain of a heavy chain. This connection is carried out via a short linker, whereas it is also possible that the domains are connected directly without a linker at all (linker of 0 AS).

Both monomers form a dimer, preferred via a disulfide bond between both constant domains of a light chain.

One of the characteristics of the invention is the fact that each of the two antigen-binding sites is formed by both monomers. The short linker of the invention does not allow an association of the variable domain of a light chain and a variable domain of a heavy chain of the same monomer, like for example in scFv. Therefore the variable domain of a light chain of one monomer forms an antigen binding site with the variable domain of a heavy chain of the other monomer.

The format of the invention is surprisingly superior compared to all formats known in the state of art.

The special combination of domains and linkers of the invention surprisingly leads to synergistic effects. It was not expected that the format of the invention could overcome the problems in the state of art.

"Polypeptides", as used herein, includes all polypeptides as described below. The basic structure of polypeptides is well known and has been described in innumerable textbooks and other publications in the art. In this context, the term is used herein to refer to any peptide or protein comprising amino acids joined to each other in a linear chain by peptide bonds. As used herein, the term refers to both short chains, which also commonly are referred to in the art as peptides, oligopeptides and oligomers, for example, and to longer chains, which generally are referred to in the art as proteins, of which there are many types.

It will be appreciated that polypeptides often contain amino acids other than the 20 amino acids at commonly referred to as the 20 naturally occurring amino acids, and that many amino acids, including the terminal amino acids, may be modified in a given polypeptide, either by natural processes such as glycosylation and other post-translational modifications, or by chemical modification techniques which are well known in the art. Common modifications include glycosylation, lipid attachment, sulfation, gamma-carboxylation of glutamic acid residues, hydroxylation and ADP-ribosylation, and these and others are described in most basic texts, such as, for instance, I. E. Creighton, Proteins-Structure and Molecular Properties, 2nd Ed., W. H. Freeman and Company, New York, 1993. Many detailed reviews are available on this subject, such as, for example, those provided by Wold, F., in Posttranslational Covalent Modification of Proteins, B. C. Johnson, Ed., Academic Press, New York, pp 1-12, 1983; Seifter et al., Meth. Enzymol. 182: 626-646, 1990 and Rattan et al., Protein Synthesis: Posttranslational Modifications and Aging, Ann. N.Y. Acad. Sci. 663: 48-62, 1992.

It will be appreciated, as is well known, and as noted above, that polypeptides are not always entirely linear. For instance, polypeptides may be branched as a result of ubiquitination, and they may be circular, with or without branching, generally as a result of posttranslational events, including natural processing events and events brought about by human manipulation which do not occur naturally. Circular, branched and branched circular polypeptides may be synthesized by non-translational natural processes and by entirely synthetic methods, as well.

Modifications can occur anywhere in a polypeptide, including the peptide backbone, the amino acid side-chains and the amino or carboxyl termini. In fact, blockage of the amino or carboxyl group in a polypeptide, or both, by a covalent modification, is common in naturally occurring and synthetic polypeptides and such modifications may be present in polypeptides of the present invention, as well. For instance, the amino terminal residue of polypeptides made in E. coli, prior to proteolytic processing, almost invariably will be N-formylmethionine.

The modifications that occur in a polypeptide often will be a function of how it is made. For polypeptides made by expressing a cloned gene in a host, for instance, the nature and extent of the modifications in large part will be determined by the host cell posttranslational modification capacity and the modification signals present in the polypeptide amino acid sequence. For instance, as is well known, glycosylation often does not occur in bacterial hosts such as E. coli. Accordingly, when glycosylation is desired, a polypeptide should be expressed in a glycosylating host, generally a eukaryotic cell.

In general, as used herein, the term polypeptide encompasses all such modifications, particularly those that are present in polypeptides synthesized by expressing a polynucleotide in a host cell.

In a preferred embodiment of the invention, the polypeptide is an antigen-binding peptide.

As used herein, the "antigen-binding" refers to that portion of a polypeptide and/or antibody which contains the amino acid residues that interact with an antigen and confer on the antibody its specificity and affinity for the antigen.

The term "antigen-binding polypeptide" as used herein means a polypeptide or protein which, according to the invention, specifically binds to an antigen.

The term "specific binding", "binding specificity,"as used herein means that the polypeptide of the present invention does not show any significant binding other molecules. Usually this binding is reversible and/or non-covalent.

The term "heavy chain" or "light chain" as used herein means the conventional antibody polypeptide heavy chain (H) or light chain (L), respectively.

The term "variable region" or "variable domain" as used herein means the conventional antibody variable region or variable domain or variable fragment (V or Fv) which confers antigen binding specificity. Variable regions employed in the invention may be derived from any germline or rearranged variable domain, or may be a synthetic variable domain based on consensus sequences of known variable domains. Usually the variable region of the heavy chain (V_{H}) and the variable region of the light chain (V_{L}) each comprise three complementarity determining regions CDR1, CDR2 and CDR3.

The term "single chain variable fragment" or "single-chain antibody" or "single-chain fragment" as used herein means any polypeptide comprising the variable region of a heavy chain, the variable region of a light chain and a linking peptide between the variable region of the heavy and the light chain, resulting in a single chain Fv or "scFv".

In terms of the invention a "domain" of a polypeptide or protein is a discrete part of an amino acid sequence that can be equated with a particular function.

In terms of the invention "antibody" or "antibody construct" refers to an intact antibody, or a fragment thereof. An antibody or antibody construct, is said to specifically bind an antigen when the dissociation constant is less than or equal to 1 µM, preferably less than or equal to 100 nM and most preferably less than or equal to 10 nM. Binding can be measured by methods known to those skilled in the art. Antibody constructs comprise a portion of an intact antibody, preferably the antigen binding or variable region of the intact antibody.

It was very surprising that a small linker consisting of 12 or less amino acids can be used to connect V_{L} and V_{H}. The use of a small linker prevents the assembling of V_{L} and V_{H} within the same chain. Thus each antigen binding site is formed by V_{L} of one chain and V_{H} of the other chain. Surprisingly these antigen binding sites lead to a higher affinity. Also this conformation is more stable compared to antibody constructs with longer linker sequences. Antibodies are targets for enzymatic cleavage, which is a major problem for antibody stability. It was very surprising that the use of the short linker of the invention results in a conformation that protects the antibody construct from enzymatic cleavage, especially from proteases.

It was not expected that antibody construct without a linker between VL and VH can be used to solve the technical problem. Therefore it was very surprising that the use of a construct without a linker showed good results in stability and specificity.

Surprisingly the polypeptides of the invention showed an additive increase of stability in comparison with all antibody formats known in the state of art, especially diabodies or (scFv-C_{K})2 antibody constructs.

Another advantage of the invention is the high expression rates of the polypeptides in plants. Therefore the production of these polypeptides is less expensive compared to the state of art.

Small antibody constructs like scFv or Fab fragments are monovalent, which is one of their major limitations. However the antigen-binding polypeptides and/or the antibody constructs of the invention are bivalent, which leads to an increased affinity and thereby a better applicability for therapeutic and/or diagnostic applications.

Utilizing linker sequences for the construction of scFv dimers was one approach to overcome these problems in the state of art. However, such dimers are formed by noncovalent association and therefore often dissociate which results in a poor stability.

Surprisingly the antibody constructs of the invention showed an exceeding stability, which is advantageous in many ways. A high stability is preferable because lower doses can be administered. Therefore a therapy with the antibody constructs of the invention is less expansive compared to the state of art.

The constructs of the invention lead to an increased performance and have an improved reliability and quality. The saving of time, material, work steps, costs and resources are also advantages of the invention. The production of the polypeptides of the invention is distinguished by a better yield and a price-reduction. The antibody construct itself shows a surprisingly increased efficiency.

Misfolding or poor stability can result in fractional populations of non-functional material and/or antibodies with the tendency to form large soluble aggregates which are potentially dangerous or immunogenic when used therapeutically. Other stability problems include impeded refolding, degradation, impaired avidity, aggregation, or loss of activity following storage.

Unstable polypeptides suffer from many problems, including unsuitability for scale-up production in bioreactors (e.g., because of low yield, significant levels of unwanted byproducts such as unassembled product, and/or aggregated material), difficulties in purification, and unsuitability for pharmaceutical preparation and use (e.g., owing to significant levels of breakdown product, poor product quality, and/or unfavourable pharmacokinetic properties). Especially instability within the variable domains of antibodies, Fabs, Fvs, or scFvs may result in many of these problems. scFv constructs in particular have demonstrated problems with stability, solubility, expression, aggregation, breakdown products, and overall manufacturability. Additionally, incorporation of scFv molecules into otherwise stable recombinant antibody products often imparts these generally undesirable traits to the new recombinant design.

It was not expected that the embodiments of the invention lead to antibody constructs stable enough to overcome the problems mentioned above.

It is known that the constant domains of a heavy chain are important for the good stability. Surprisingly the polypeptides and/or antibody formats of the invention are extremely stable without having a constant domain of a heavy chain.

It was very surprising the addition of constant domains of a light chain led to superior results.

The new antigen-binding polypeptide and/or the antibody construct can be used in the treatment, prophylaxes, aftercare, follow-up treatment or diagnosis for all disease that can be treated with antibodies.

Diseases in terms of the invention are viral infectious diseases, bacterial infectious diseases, parasitic infectious diseases, fungal infectious diseases, prion infectious diseases, tumor diseases and/or autoimmune diseases.

All diseases that are caused by an infections agent or a pathogen can be treated and/or prevented with the polypeptides and/or antibody constructs of the invention.

As used herein, an "infectious agent" and "pathogen" denotes both microbes and parasites. A "microbe" includes viruses, bacteria, rickettsia, mycoplasma, protozoa, fungi and like microorganisms. A "parasite" denotes infectious, generally microscopic or very small multicellular invertebrates, or ova or juvenile forms thereof, which are susceptible to antibody-induced clearance or lytic or phagocytic destruction, such as malarial parasites, spirochetes, and the like. Examples of infectious agents include, for example, a fungus, virus, parasite, bacterium, protozoan, or mycoplasm.

For example the fungus may be from the species of Microsporum, Trichophyton, Epidermophyton, Sporothrix schenckii, Cyrptococcus neoformans, Coccidioides immitis, Histoplasma capsulatum, Blastomyces dermatitidis, or Candida albicans.

For example the virus may be from the species of human immunodeficiency virus (HIV), herpes virus, cytomegalovirus, rabies virus, influenza virus, hepatitis B virus, SARS coronavirus, Sendai virus, feline leukemia virus, Reo virus, polio virus, human serum parvo-like virus, simian virus 40, respiratory syncytial virus, mouse mammary tumor virus, Varicella-Zoster virus, Dengue virus, rubella virus, measles virus, adenovirus, human T-cell leukemia viruses, Epstein-Barr virus, murine leukemia virus, mumps virus, vesicular stomatitis virus, Sindbis virus, lymphocytic choriomeningitis virus, wart virus and blue tongue virus.

For example the bacterium may be, for example, Anthrax bacillus, Streptococcus agalactiae, Legionella pneumophilia, Streptococcus pyogenes, Escherichia coli, Neisseria gonorrhoeae, Neisseria meningitidis, Pneumococcus, Hemophilis influenzae B, Treponema pallidum, Lyme disease spirochetes, Pseudomonas aeruginosa, Mycobacterium tuberculosis, Mycobacterium leprae, Brucella abortus, and Tetanus toxin.

For example the parasite may be a helminth or a malarial parasite.

For example the protozoan may be Plasmodium falciparum, Plasmodium vivax, Toxoplasma gondii, Trypanosoma rangeli, Trypanosoma cruzi, Trypanosoma rhodesiensei, Trypanosoma brucei, Schistosoma mansoni, Schistosoma japanicum, Babesia bovis, Eimeria tenella, Onchocerca volvulus, Leishmania tropica, Trichinella spiralis, Onchocerca volvulus, Theileria parva, Taenia hydatigena, Taenia ovis, Taenia saginata, Echinococcus granulosus or Mesocestoides corti. The mycoplasma may be Mycoplasma arthritidis, Mycoplasma hyorhinis, Mycoplasma orale, Mycoplasma arginini, Acholeplasma laidlawii, Mycoplasma salivarum, and Mycoplasma pneumoniae.

The use of the polypeptides and/or the antibody constructs of the invention is especially advantageous for AIDS, AIDS related complex, chickenpoxx, common cold, cytomegalovirus infection, colorado tick fever - dengue fever, ebola hemorrhagic fever, hand, foot and mouth disease, hepatitis, herpes simplex, herpes zoster, HPV, influenza, lassa fever, measles, marburg hemorrhagic fever, infectious mononucleosis, mumps, norovirus, poliomyelitis, Progressive multifocal leukencephalopathy, rabies, rubella, smallpox, viral encephalitis, viral gastroenteritis, viral meningitis, viral pneumonia, West nile disease, yellow fever, anthrax, bacterial meningitis, botulism, brucellosis, campylobacteriosis, cat scratch disease, cholera, diphtheria, epidemic typhus, gonorrhea, impetigo, legionellosis, leprosy (hansen's disease), leptospirosis, listeriosis, lyme disease, melioidosis, rheumatic fever, MRSA infection, nocardiosis, pertussis (whooping cough), plague, pneumococcal pneumonia, psittacosis, Q fever, Rocky mountain spotted fever (RMSF), salmonellosis, scarlet fever, shigellosis, syphilis, tetanus, trachoma, tularemia, typhoid Fever, typhus, urinary tract infections, african trypanosomiasis, amebiasis, ascariasis, babesiosis, chagas disease, clonorchiasis, cryptosporidiosis, cysticercosis, diphyllobothriasis, dracunculiasis, echinococcosis, enterobiasis, fascioliasis, fasciolopsiasis, filariasis, free-living amebic infection, giardiasis, gnathostomiasis, hymenolepiasis, isosporiasis, kala-azar, leishmaniasis, malaria, metagonimiasis, myiasis, onchocerciasis, pediculosis, pinworm Infection, Severe acute respiratory syndrome (SARS), scabies, schistosomiasis, taeniasis, toxocariasis, toxoplasmosis, trichinellosis, trichinosis, trichuriasis, trichomoniasis, tuberculosis, trypanosomiasis, aspergillosis, blastomycosis, candidiasis, coccidiosis. coccidioidomycosis, cryptococcosis, histoplasmosis, tinea pedis, transmissible spongiform encephalopathy, bovine spongiform encephalopathy, creutzfeldt-Jakob disease, alpers Syndrome, acute disseminated encephalomyelitis, addison's disease, ankylosing spondylitis, antiphospholipid antibody syndrome, autoimmune hemolytic anemia, autoimmune hepatitis, autoimmune inner ear disease, bullous pemphigoid, coeliac disease, chagas disease, chronic obstructive pulmonary disease, dermatomyositis, diabetes mellitus type 1, endometriosis, goodpasture's syndrome, graves' disease, guillain-barré syndrome, hashimoto's disease, hidradenitis suppurativa, idiopathic thrombocytopenic purpura, interstitial cystitis, lupus erythematosus, morphea, multiple sclerosis, myasthenia gravis, narcolepsy, neuromyotonia, pemphigus vulgaris, pernicious anaemia, polymyositis, primary biliary cirrhosis, rheumatoid arthritis, schizophrenia, scleroderma, sjögren's syndrome, temporal arteritis, vasculitis, vitiligo, wegener's granulomatosis, leukemia, adrenocortical carcinoma, basal cell carcinoma, bile duct cancer, extrahepatic, bladder cancer, bone cancer, osteosarcoma, brain stem glioma, brain tumor, breast cancer, bronchial adenomas, burkitt lymphoma, carcinoma, colon cancer , esophageal cancer, gallbladder cancer, gastric cancer, gastrointestinal stromal tumor (GIST), glioma, hepatocellular cancer, hodgkin lymphoma, renal cell cancer, liver cancer, lung cancer, lymphomas, melanoma, mesothelioma, neuroblastoma, non-hodgkin lymphoma, oral cancer, ovarian cancer, pancreatic cancer, prostate cancer, rectal cancer, salivary gland cancer, testicular cancer and/or urethral cancer.

The increasing number of antibiotic-resistant bacteria emphasizes the need to find alternatives. Surprisingly, the antigen-binding polypeptides and/or antibody constructs can also be used as a complementary treatment against pathogens that are difficult to treat with traditional antibiotics.

Surprisingly the antibody construct and/or the antigen-binding polypeptide of the invention can also be advantageously used in all in vitro diagnostic methods, where their use minimizes cross-reactivity and false positive and negative results.

In terms of the invention, the antigen-binding polypeptide and/or the antibody construct can be constructed in a way they are able to bind a certain antigen.

A person skilled in the art knows how to adapt the CDR regions of the polypeptide and/or the antibody construct of the invention in a way in binds to a given antigen.

Any antigen-binding polypeptide with the structure explained above is an antibody construct of the invention, unregarded the antigen specificity.

While the invention has been described in terms of preferred embodiments, the skilled artisan will appreciate that various modifications, substitutions, omissions and changes may be made without departing from the spirit thereof. Accordingly, it is intended that the scope of the present invention be limited solely by the scope of the claims of the invention, including equivalents thereof. Therefore, as will be apparent to those skilled in the art to which the invention is addressed, the present invention may be embodied in forms other than those specifically disclosed without departing from the spirit or essential characteristics of the invention. The particular embodiments of the present invention as described are therefore to be considered in all respects as illustrative and not restrictive. The scope of the present invention is as set forth in the appended claims rather than being limited to the examples contained in the foregoing description.

It should be understood that various alternatives to the embodiments of the invention described herein may be employed in practicing the invention. It is intended that the claims of the invention define the scope of the invention and that the method and apparatus within the scope of these claims and their equivalents be covered thereby.

Those skilled in the art will therefore understand all those molecules as equivalent which he may apply without this having an effect on the solution of the problem of the invention. They know that the claimed sequences must not be modified by changes, substitutions or deletions in a way that they cannot be used for solving the problem of the invention.

In another preferred embodiment the present invention relates to the polypeptide and/or the antibody construct, wherein both constant domains of a light chain are kappa domains. Surprisingly the use of kappa chains resulted in high bioactivity which leads to an exceeding effectivity. The use of kappa chains is also advantageous because the transient expression of these antibody constructs is surprisingly high.

Also preferred is the polypeptide and/or the antibody construct, wherein both constant domains of a light chain are lambda domains.

The use of lambda chains resulted in a surprisingly high activity *in vitro* and *in vivo.* A high activity is especially advantageous for a therapeutical application, because a lower dose can be used, which prevents side effects.

Also preferred is the polypeptide and/or the antibody construct of the invention, wherein the constant domains are connected, preferred covalently, especially preferred via a disulfide bridge. Dimers that are formed by non-covalent association often dissociate; the invention solves this problem by introducing a disulfide bond. Analyses of molecular forms proved that the polypeptides and/or antibodies of the invention do not dissociate and therefore are dimeric. Surprisingly the disulfide bridge leads to a much higher stability, especially proteolytic stability, of the fragment. The antigen-binding polypeptide and/or the antibody construct bearing a disulfide bridge are more resistant to for example enzymatic cleavage. This leads to a higher half-life in an organism, which results in a higher efficacy.

Poor stability can affect the ability of an antibody or antibody domain to fold properly when expressed in various cellular systems, e.g., in bacterial or mammalian expression systems.

Also preferred is the polypeptide and/or the antibody construct of the invention, wherein the constant domains are connected via hydrophobic interaction. Hydrophobic interaction can be superior to covalent associations under certain circumstances. A person skilled in the art knows that there are several ways to connect two chains of a polypeptide. Disulfide bridges and hydrophobic interactions are preferred embodiments of the invention, however it is understood that any kind of connection, covalent or non- covalent, known in the state of art can be used to connect the polypeptide of the invention. A skilled person knows that which connection or association is suited best for a certain condition.

Another preferred embodiment of the invention is the polypeptide and/or the antibody construct, wherein said linker is not susceptible to protease cleavage.

In the state of art the linkers in antibody constructs are often a target for protease cleavage. The special linkers of the invention are resistant to enzymatic cleavage, especially protease cleavage. Especially preferred is a linker, comprising the amino acid sequence of Seq. ID NO 41 (GGSGGS). The use of this linker resulted in an extremely high stability of the antibody construct. The antibody constructs of the invention are more stable and therefore superior compared to antibodies in the state of art.

As is well-known to those skilled in the art, some amino acids have analogous physicochemical properties so that these amino acids advantageously can be replaced by each other. For example, these include the group of nonpolar (hydrophobic) amino acids (a) glycine, alanine, valine, leucine and/or isoleucine; or the hydroxy amino acids (b) serine, threonine and/or tyrosine, the amides of amino dicarboxylic acids (c) asparagine and glutamine, the amino dicarboxylic acids (d) aspartic acid and glutamic acid; the basic amino acids (e) lysine, arginine and/or ornithine as well as the group of aromatic amino acids (f) phenylalanine, tyrosine and/or tryptophan. Another aspect is the replacement of amino acids by structural similar amino acids. For example this is the case in the group with a β-functional group (g) cysteine, methionine, serine, α- aminobutyric acid and selenocysteine as well as the turn-inducing group (h) praline, 1-amino-2-carboxy cyclohexane, pipecolic acid and an ortho-aminobenzoic acid. Amino acids within one and the same group (a-h) can be replaced with one another. In all cases, peptide sequences will have a sufficient homology to be an analogous to an amino acid sequence of the peptides of the invention. Furthermore, the amino acids can be replaced by modified amino acids or specific enantiomers. Further modifications are possible in accordance with the teaching of WO 99/62933 or WO 02/38592. It is obviously known to a person skilled in the art that it is possible to provide functionally analogous means for the peptides and nucleic acids of the invention. In the meaning of the invention, "functionally analogous means" are means which are functionally equivalent or equivalent to the peptides and nucleic acids of the invention. In order to check whether a sequence is functionally equivalent (= equivalent) to the peptides or nucleic acids of the invention, one must check whether the functionally analogous/equivalent sequence of the peptide or of the nucleic acid produces essentially the same function in essentially the same way with essentially the same outcome. That is, the outcome produced with the peptides or nucleic acids of the invention may also be produced with the functionally analogous/equivalent means. A person skilled in the art can therefore easily and quickly verify whether a functionally analogous/equivalent sequence provided by them is covered by the invention. The functionally analogous sequence is covered by the invention if it may be used for solving the problem of the invention and if the solving of the problem of the invention can be carried out in essentially the same way as is claimed by the invention.

In another advantageous embodiment of the invention the amino acid molecule has at least 60%, preferably 70%, more preferably 80%, especially preferably 90% homology to the amino acid molecule of Seq. ID NO 41.

Also preferred is the use of the linker of the invention for the preparation of an antibody construct. Surprisingly this linker can not only be used in the antibody constructs of the invention but also in other antibody constructs known in the state of art. It was very surprising that by the use of the linker in known antibody constructs, these constructs become more stable, more specific and therefore more effective.

In another preferred embodiment the invention relates to the polypeptide and/or the antibody construct, wherein said polypeptide and/or antibody construct is monospecific.

In another preferred embodiment the present invention relates to the polypeptide and/or the antibody construct, wherein said polypeptide and/or an antibody construct is bispecific. Preferred is a bispecific protein dimer, comprising one monomer, wherein said V_{H} domain of specificity A is linked to a light chain of specificity B, and the second monomer, wherein said V_{H} domain of specificity B is linked to a light chain of specificity A.

If a monospecific or a bispecific antibody construct is suited for the application depends on the target. One benefit of the bispecific antibody construct is that it is possible for such an antibody to simultaneously bind two different antigens, for example a bispecific antibody can bind to a cytotoxic cell and a target cell at the same time.

Monospecific antibodies bind to the same antigen sequence with both antigen binding sites; therefore they can neutralize or eliminate the antigen in a very effective way.

The use of monospecific antibodies is also preferred in diagnostics'.

Also preferred is the polypeptide and/or the antibody construct, wherein said linker is selected from the group consisting of a GS-linker, a yol-linker and/or a linker with the amino acid sequence GGSGGS.

There is no restriction site in the GGSGGS linker, which helps to prevent enzymatic cleavage. The GGSGGS linker is relatively inflexible, which is advantageous for the conformation of the polypeptide and/or antibody construct of the invention.

It is understood, that a person skilled in the art knows that certain amino acids can be replaced by others.

Another preferred embodiment of the invention is the polypeptide and/or the antibody construct, wherein the polypeptide and/or an antibody construct is a non-immunoglobulin polypeptide.

A non-immunoglobulin polypeptide does not have an Fc region and is therefore much smaller compared to a full length antibody. Antibodies without an Fc region are superior for the use in diagnostics, because they do not interact with Fc receptors and therefore cause a decreased background staining.

Additionally the lower molecular mass compared to a full-length antibody is associated with increased expression rates.

Another preferred embodiment of the invention is the polypeptide and/or the antibody construct, comprising the amino acid sequence selected from the group comprising
Seq. ID NO 1 Seq. ID NO 2 and/or Seq. ID NO 3

Antibody constructs comprising sequence ID NO 1 showed especially good results in experiments with Eimeria. A dimer consisting of two chains, each comprising Seq. ID NO 1 resulted in a high activity against the Eimeria antigen in vitro.

Antibody constructs comprising sequence ID NO 2 and 3 exhibited a good activity against HIV. The expression levels of dimers consisting of two chains, each comprising Seq. ID NO 2 and/or 3 were surprisingly high. Additionally antibody constructs comprising Seq. ID NO 3 were extremely stable, even under reducing conditions most of the polypeptides were present in the dimeric form.

Another preferred embodiment of the invention is the polypeptide and/or an antibody construct, wherein at least one variable domain of a heavy chain V_{H} comprises the amino acid sequence selected from the group comprising Seq. ID NO 4 Seq. ID NO 5 and/or Seq. ID NO 6

The use of Seq. ID NO 4 in an antibody construct led to a high specificity against the Eimeria antigen, whereas the use of Seq. ID NO 5 and 6 was superior for the binding of HIV.

Also preferred is the polypeptide and/or an antibody construct, wherein at least one variable domain of a light chain V_{L} comprises the amino acid sequence selected from the group comprising Seq. ID NO 7 Seq. ID NO 8 and/or Seq. ID NO 9

The V_{L} domain comprising Seq. ID NO 7 was advantageous in antibody constructs directed against Eimeria. V_{L} domains comprising Seq. ID NO 8 and/or Seq. ID NO 9 can be used successfully in antibody constructs against HIV.

The sequences ID NO 2, 3, 5, 6, 8 and 9 were designed based on the disclosure of Trkola et al 1996 (Human monoclonal antibody 2G12 defines a distinctive neutralization epitope on the gp120 glycoprotein of human immunodeficiency virus type I. J. Virol. 70:1100-1108), Kunert R., Rueker F., and Katinger H. 1998 (Molecular Characterization of Five Neutralizing Anti-HIV Type 1 Antibodies: Identification of Nonconventional D Segments in the Human Monoclonal Antibodies 2G12 and 2F5. Aids Resesearch and human retroviruses. Vol 14(13):1115-1128) and Purtschner et al 1994 (A broadly neutralizing human monoclonal antibody against gp41 of human immunodeficiency virus typ I. AIDS RESearch and HUMAN RETROVIRUSES Vol 10:1651-1658)

In another preferred embodiment of the invention the polypeptide and/or an antibody construct, is a polypeptide and/or antibody construct wherein at least one, preferred two constant domain of a light chain C_{L} comprises the amino acid sequence selected from the group comprising Seq. ID NO 10

Surprisingly C_{L} domains with the amino acid sequence of Seq. ID NO 10 showed very good results. The stability of antibody constructs with two C_{L} domains comprising Seq. ID NO 10 was superior to other constructs. Advantageous is also the high binding affinity of these construct to various antigens, for example antigens associated with Eimeria, HIV, SARS or Tuberculosis. Another preferred embodiment is the polypeptide and/or an antibody construct wherein at least one variable domain of a heavy chain V_{H} comprises a V_{H} CDR1, wherein V_{H} CDR1 comprises an amino acid sequence selected from the group comprising Seq. ID NO 11 (DHAIH), Seq. ID NO 12 (VYIIH), Seq. ID NO 13 (SYAMS), Seq. ID NO 14 (NFWMH), Seq. ID NO 15 (NFWIH) and/or Seq. ID NO 16 (DTYIH).

Also preferred is the polypeptide and/or an antibody construct, wherein at least one variable domain of a heavy chain comprises a V_{H} CDR2, wherein V_{H} CDR2 comprises an amino acid sequence selected from the group comprising Seq. ID NO 17 (YFSPGNNDIKYNEKFKG), Seq. ID NO 18 (YINPYNDGTKYNEKFRG), Seq. ID NO 19 (SISSGGSTYYPDSVKG), Seq. ID NO 20 (EINPSNGDTNYNEKFKR), Seq. ID NO 21 (EINPSNGDTKYNEKFRR) and/or Seq. ID NO 22 (RIDPANGNTKYDPKFQG).

Also preferred is the polypeptide and/or an antibody construct, wherein at least one variable domain of a heavy chain comprises a V_{H} CDR3, wherein V_{H} CDR3 comprises an amino acid sequence selected from the group comprising Seq. ID NO 23 (WPGAMDY), Seq. ID NO 24 (FYYGSSSVFDY), Seq. ID NO 25 (NYGSYWGDSYFFDY), Seq. ID NO 26 (RGWLHFWYFDV) and/or Seq. ID NO 27 (SLGDYFAMNY).

In another preferred embodiment the invention relates to the polypeptide and/or an antibody construct, wherein at least one variable domain of a light chain comprises a V_{L} CDR1, wherein V_{L} CDR1 comprises an amino acid sequence selected from the group comprising Seq. ID NO 28 (KASQDVGTAVA), Seq. ID NO 29 (KASQNVGTNVA), Seq. ID NO 30 (RSSQSLENSNGN TYLN) and/or Seq. ID NO 31 (RASGNIHNYLA).

Also preferred is the polypeptide and/or an antibody construct, wherein at least one variable domain of a light chain comprises a V_{L} CDR2, wherein V_{L} CDR2 comprises an amino acid sequence selected from the group comprising Seq. ID NO 32 (WASTRHT), Seq. ID NO 33 (SASYRYS), Seq. ID NO 34 (KVSNRFS), and/or Seq. ID NO 35 (NAKTLAD).

Also preferred is the polypeptide and/or an antibody construct, wherein at least one variable domain of a light chain comprises a V_{L} CDR3, wherein V_{L} CDR3 comprises an amino acid sequence selected from the group comprising Seq. ID NO 36 (QQYSSYPLT), Seq. ID NO 37 (QQYNSYPLT), Seq. ID NO 38 (HQYSSYPLT), Seq. ID NO 39 (FQGSHVPYT) and/or Seq. ID NO 40 (QHFWSTPPT).

Preferred is the polypeptide and/or an antibody construct, wherein at least one variable domain of a heavy chain comprises a V_{H} CDR1, a V_{H} CDR2 and/or a V_{H} CDR3, wherein V_{H} CDR1 comprises Seq. ID NO 11, V_{H} CDR2 comprises Seq. ID NO 17 and/or V_{H} CDR3 comprises Seq. ID NO 23 and wherein at least one variable domain of a light chain comprises a V_{L} CDR1, a V_{L} CDR2 and/or a V_{L} CDR3, wherein V_{L} CDR1 comprises Seq. ID NO 28, V_{L} CDR2 comprises Seq. ID NO 32 and/or V_{L} CDR3 comprises Seq. ID NO 36.

Preferred is the polypeptide and/or an antibody construct, wherein at least one variable domain of a heavy chain comprises a V_{H} CDR1, a V_{H} CDR2 and/or a V_{H} CDR3, wherein V_{H} CDR1 comprises Seq. ID NO 12, V_{H} CDR2 comprises Seq. ID NO 18 and/or V_{H} CDR3 comprises Seq. ID NO 24 and wherein at least one variable domain of a light chain comprises a V_{L} CDR1, a V_{L} CDR2 and/or a V_{L} CDR3, wherein V_{L} CDR1 comprises Seq. ID NO 29, V_{L} CDR2 comprises Seq. ID NO 33 and/or V_{L} CDR3 comprises Seq. ID NO 37.

Preferred is the polypeptide and/or an antibody construct, wherein at least one variable domain of a heavy chain comprises a V_{H} CDR1, a V_{H} CDR2 and/or a V_{H} CDR3, wherein V_{H} CDR1 comprises Seq. ID NO 13, V_{H} CDR2 comprises Seq. ID NO 19 and/or V_{H} CDR3 comprises Seq. ID NO 25 and wherein at least one variable domain of a light chain comprises a V_{L} CDR1, a V_{L} CDR2 and/or a V_{L} CDR3, wherein V_{L} CDR1 comprises Seq. ID NO 29, V_{L} CDR2 comprises Seq. ID NO 33 and/or V_{L} CDR3 comprises Seq. ID NO 37.

Preferred is the polypeptide and/or an antibody construct, wherein at least one variable domain of a heavy chain comprises a V_{H} CDR1, a V_{H} CDR2 and/or a V_{H} CDR3, wherein V_{H} CDR1 comprises Seq. ID NO 14, V_{H} CDR2 comprises Seq. ID NO 20 and/or V_{H} CDR3 comprises Seq. ID NO 26 and wherein at least one variable domain of a light chain comprises a V_{L} CDR1, a V_{L} CDR2 and/or a V_{L} CDR3, wherein V_{L} CDR1 comprises Seq. ID NO 28, V_{L} CDR2 comprises Seq. ID NO 32 and/or V_{L} CDR3 comprises Seq. ID NO 38.

Preferred is the polypeptide and/or an antibody construct, wherein at least one variable domain of a heavy chain comprises a V_{H} CDR1, a V_{H} CDR2 and/or a V_{H} CDR3, wherein V_{H} CDR1 comprises Seq. ID NO 15, V_{H} CDR2 comprises Seq. ID NO 21 and/or V_{H} CDR3 comprises Seq. ID NO 26 and wherein at least one variable domain of a light chain comprises a V_{L} CDR1, a V_{L} CDR2 and/or a V_{L} CDR3, wherein V_{L} CDR1 comprises Seq. ID NO 30, V_{L} CDR2 comprises Seq. ID NO 34 and/or V_{L} CDR3 comprises Seq. ID NO 39.

Preferred is the polypeptide and/or an antibody construct, wherein at least one variable domain of a heavy chain comprises a V_{H} CDR1, a V_{H} CDR2 and/or a V_{H} CDR3, wherein V_{H} CDR1 comprises Seq. ID NO 16, V_{H} CDR2 comprises Seq. ID NO 22 and/or V_{H} CDR3 comprises Seq. ID NO 27 and wherein at least one variable domain of a light chain comprises a V_{L} CDR1, a V_{L} CDR2 and/or a V_{L} CDR3, wherein V_{L} CDR1 comprises Seq. ID NO 31, V_{L} CDR2 comprises Seq. ID NO 35 and/or V_{L} CDR3 comprises Seq. ID NO 40.

In another advantageous embodiment of the invention the polypeptides comprises sequence, wherein said sequence has at least 60%, preferably 70%, more preferably 80%, especially preferably 90% homology to the amino acid molecule of any of the Seq. ID NO 1 to Seq. ID NO 40.

In another preferred embodiment the invention relates to the polypeptide and/or an antibody construct, wherein the polypeptide binds to Eimeria, preferred Eimeria tenella, HIV, Hepatitis B, SARS coronavirus, Mycobacterium tuberculosis and/or Mycobacterium leprae.

Also preferred is the polypeptide and/or the antibody construct, which neutralizes or ameliorates the pathogenic effects of a pathogen, preferred *Eimeria,* Eimeria tenella, HIV, Hepatitis B, SARS coronavirus, Mycobacterium tuberculosis and/or Mycobacterium leprae.

Also preferred is the polypeptide and/or the antibody construct, which interferes with invasion, establishment or propagation of *Eimeria* species in birds, preferred poultry, more preferred chicken.

Eimeria is a parasite responsible for the poultry disease coccidiosis. Medications are available for the parasite; however, they are expensive and drug resistance is common as well as possible drug residues in the meat once the animal is butchered. Symptoms of *Eimeria* infection include bloody diarrhea due to intestinal epithelium dying off since a large number of oocysts and merozoites burst out of the cells. Necrotic tissue clogs the cecum causing the organ to die. There is no satisfying treatment of coccidosis so far, which is a major problem for the poultry industry. Therefore Eimeria is a preferred target for the antibody construct of the invention.

Another preferred embodiment of the invention is a polynucleotide comprising a nucleotide sequence which encodes a polypeptide and/or an antibody construct mentioned above. Polynucleotide(s) generally refers to any polyribonucleotide or polydeoxyribonucleotide, which may be unmodified RNA or DNA or modified RNA or DNA. Thus, for instance, polynucleotides as used herein refers to, among others, single- and double-stranded DNA, DNA that is a mixture of single- and double-stranded regions, single- and double-stranded RNA, and RNA that is mixture of single- and double-stranded regions, hybrid molecules comprising DNA and RNA that may be single-stranded or, more typically, double-stranded or a mixture of single- and double-stranded regions.

As used herein, the term "polynucleotide" includes DNAs or RNAs as described above that contain one or more modified bases. Thus, DNAs or RNAs with backbones modified for stability or for other reasons are "polynucleotides" as that term is intended herein. Moreover, DNAs or RNAs comprising unusual bases, such as inosine, or modified bases, such as tritium-labelled bases, to name just two examples, are polynucleotides as the term is used herein. It will be appreciated that a great variety of modifications have been made to DNA and RNA that serve many useful purposes known to those of skill in the art. The term "polynucleotide" as it is employed herein embraces such chemically, enzymatically or metabolically modified forms of polynucleotides, as well as the chemical forms of DNA and RNA characteristic of viruses and cells, including simple and complex cells, inter alia.

In another preferred embodiment the invention relates to a nucleotide construct comprising one of the polynucleotides mentioned above, wherein said polynucleotide is operably linked to a promoter that drives expression in a host cell.

The term "promoter" refers to a DNA sequence which directs the transcription of a structural gene to produce mRNA. Typically, a promoter is located in the 5' region of a gene, proximal to the start codon of a structural gene. If a promoter is an inducible promoter, then the rate of transcription increases in response to an inducing agent. In contrast, the rate of transcription is not regulated by an inducing agent if the promoter is a constitutive promoter.

Another preferred embodiment of the invention relates to vector comprising one of the mentioned polynucleotide or the nucleotide construct.

The term "vector" includes a nucleic acid molecule capable of transporting another nucleic acid to which it has been linked including, but not limited to, plasmids and viral vectors. Certain vectors are capable of autonomous replication in a host cell into which they are introduced while other vectors can be integrated into the genome of a host cell upon introduction into the host cell, and thereby, are replicated along with the host genome. Moreover, certain vectors are capable of directing the expression of genes to which they are operably linked.

Especially preferred is a phage display vector, a bacterial expression vector, a yeast expression vector, a fungus expression vector, an algae expression vector or a plant expression vector.

In order to prepare the introduction of foreign genes into higher plants a variety of vectors are available which contain replicating signals for *E. coli* and a marker gene for selecting transformed bacterial cells. Examples for such vectors are pBR322, pUC series, M13mp series, pACYC184, pBlueSfi and the like. The desired sequence may be introduced into the vector at a suitable restriction site. The resulting plasmid is then used for the transconstruction of *E. coli* cells. Transformed *E. coli* cells are cultivated in a suitable medium and then harvested and lysed, and the plasmid is recovered. In order to characterize the produced plasmid DNA in general, restriction analysis, gel electrophoresis and further biochemical and molecular biological methods are used. After each manipulation step the plasmid DNA may be cleaved and the obtained DNA fragments may be linked to other DNA sequences.

Several known techniques are available for introducing DNA into a plant host cell, and the person skilled in the art will not have any difficulties in selecting a suitable method. These techniques comprise the transconstruction of plant cells with T-DNA by use of *Agrobacterium tumefaciens* or *Agrobacterium rhizogenes* as the transforming agent, the fusion of protoplasts, the direct gene transfer of isolated DNA into protoplasts, the electroporation of DNA, the introduction of DNA by means of the biolistic method as well as further possibilities.

For injection and electroporation of DNA into plant cells, no specific requirements for the plasmids are necessary per se. The same is true for direct gene transfer. Plain plasmids such as pUC and pBlueScript derivatives may be used. The presence of a selectable marker gene is necessary if entire plants are to be regenerated from such transformed cells. The person skilled in the art is familiar with these gene selection markers and will not have any problems in selecting a suitable one.

Further DNA sequences may be required depending on the introduction method for desired genes into the plant cell. If, for example, the Ti or Ri plasmid is used for the transconstruction of the plant cell, at least the right border, however more often both the right and the left border of the T-DNA in the Ti or Ri plasmid, has to be linked as flanking region to the genes to be introduced.

If agrobacteria are used for transconstruction, the DNA to be introduced has to be cloned into special plasmids, either into an intermediate or into a binary vector. Intermediate vectors may be integrated into the Ti or Ri plasmid of the agrobacteria by homologous recombination via sequences which are homologous to sequences in the T-DNA. This also contains the *vir* region which is required for T-DNA transfer. Intermediate vectors are not able to replicate in agrobacteria. With the aid of a helper plasmid the intermediate vector may be transferred to *Agrobacterium tumefaciens* via conjugation. Binary vectors are able to replicate in *E. coli* as well as in agrobacteria. They contain a selection marker gene, and a linker or polylinker framed by the right and left T-DNA border region. They may be transformed directly into agrobacteria. The agrobacterial host cell should contain a plasmid with a *vir* region. The *vir* region is required for the transfer of the T-DNA into the plant cell. Additional T-DNA may be present. Such a transformed agrobacterial cell is used for the transconstruction of plant cells.

The use of T-DNA for the transconstruction of plant cells has been studied intensively, and has been sufficiently described in generally known reviews and plant transconstruction manuals.

For transfer of the DNA into the plant cell, plant explants may be cultivated for this purpose together with *Agrobacterium tumefaciens* or *Agrobacterium rhizogenes*. From the infected plant material (e.g. leaf pieces, stem segments, roots, but also protoplasts or suspension-cultivated plant cells) whole plants may be regenerated in a suitable medium which may contain antibiotics or biocides for selection of transformed cells. Regeneration of plants may take place according to conventional regeneration methods by use of known growth media. The resulting plants may be analyzed for the presence of the introduced DNA. Other possibilities for the introduction of foreign DNA by use of biolistic methods or protoplast transconstruction are well known and as have been described extensively.

Once the introduced DNA has been integrated into the plant cell genome, it is generally stable there and is maintained in the progeny of the originally transformed cell as well. Usually it contains a selection marker which allows the selection of transformed cells from those lacking the introduced DNA. This selection marker endows the transformed plant cells with resistance to a biocide or an antibiotic, such as kanamycin, G418, bleomycin, hygromycin, methotrexate, glyphosate, streptomycin, sulfonylurea, gentamycin or phosphinotricin and others. But also nutritive markers and screening markers (such as GFP, green fluorescent protein) are used as alternatives to antibiotics resistance markers. It is also possible to perform this selection procedure without any selection marker, however, this requires a quite high screening expenditure.

The resulting plants may be cultivated normally, and may be crossbred with plants having the same transformed hereditary disposition or other predispositions. The resulting hybrids will have the pertinent phenotype characteristics. Seeds may be obtained from the plant cells.

Two or more generations should be generated to ensure that the phenotypic feature is stably maintained and inherited. Additionally, seeds should be harvested to verify the maintenance of the respective phenotype or other features.

In a preferred embodiment, the vector comprising the polynucleotide which encodes the antigen-binding polypeptide of the present invention, preferrd the antibody fragment or a derivative thereof, which specifically binds to *Eimeria,* contains specific regulatory elements, either constitutive or inducible either by biotic or abiotic stimuli, especially promoters, specific for the expression of the cloned construct in the prokaryotic or eukaryotic host cell, e.g. in bacteria, yeast or plant cells. For the expression of the polypeptides of the present invention in a plant cell or a microorganism cell, in principle any promoter can be considered which is active in the respective host cell. The promoter may be homologous or heterologous to the cell to be transformed.

For plant expression, the promoter is preferably tissue specific, but it may also be development-specific. The promoter may e.g. be active in tissues that are used as fodder such as seed, leaf and fruit tissues. The most preferred promoters for plant expression are seed specific promoters. Useful promoters for seed specific expression are the USP promoter, preferably the long (Zakharov et al., 2004, J Exp Bot 55: 1463-71) or the short USP promoter (Bäumlein et al., 1991, Mol Gen Genet 225: 459-67). Other promoters which can be used in the present invention are the NOS-promoter, the Vicilin promoter, the Phaseolin promoter, the Actin promoter, the Arcelin promoter, the Hordein promoter (Brandt et al., 1987, Carlsberg Res Commun 50: 333-45) and the Napin promoter, the ACP promoter as well as the FatB3- and FatB4 promoters, the 35S RNA promoter of the Cauliflower Mosaic Virus, the Ubiquitin promoter from maize, or the LeB4 promoter. The following publication summarizes most of the useful promoters: (Zakharov et al., 2004, J Exp Bot 55: 1463-71).

Useful promoters for the expression in microorganisms are e.g. the lac operon promoter, the T3/T7 promoter, and the AOX promoter.

These promoters are well known to a person skilled in the art of molecular biology. In any case the skilled person can find suitable promoters in the literature, e.g. in relevant scientific journals and gene databases, or can isolate them from any desired organism using standard methods. Leader sequences may also be introduced into the vector, e.g. in order to achieve secretion of the polypeptides of the present invention into the medium. For example, the amino acid sequence "EAYA" is a cleavage site at the C terminus of a leader sequence. The "KDEL" sequence at the C terminus of a polypeptide sequence is an endoplasmatic reticulum (ER) retention signal in plants.

The invention also relates to a host cell comprising the polynucleotide, the vector and/or the polypeptide and/or the antibody construct. The host cell can be a prokaryotic or an eukaryotic cell; preferred is a host cell selected from the group comprising a bacterial cell, a yeast cell, a fungus cell, an algae cell and/or a plant cell. Especially preferred is a host cell selected from the group comprising an *Escherichia coli* cell, a Streptomyces cell, a *Pichia pastoris* cell, a Schizosaccharomyces cell, an Aspergillus cell, a Chlamydomonas cell, a *Nicotiana spec*. cell, a *Pisum sativum* cell, a *Hordeum vulgare* cell, a *Zea mays* cell, a rapeseed cell, a *Cucumis sativus* cell, and/or a *Linum usitatissimum* cell.

Preferably, the host cells are stably or transiently transformed with the above-described vectors of the present invention.

In another preferred embodiment the invention relates to a transgenic plant, parts thereof and/or microorganisms, containing the polynucleotide, the nucleic construct, the vector and/or the polypeptide of the present invention.

As used herein, the term "transgenic" refers to cells, cell cultures, organisms, bacteria, fungi, animals, plants and progeny of any of the preceding, which have received foreign or modified nucleic acid sequence.

Preferably, the host cells or the plants or plant parts or microorganisms of the present invention express and synthesize the antigen-binding polypeptide and/or the antibody construct of the present invention. These host cells may be any prokaryotic or eukaryotic cells, preferably microorganismic cells or plant cells, more preferably bacterial, yeast, fungus, algae and plant cells. Most preferred among the microorganismic cells are *Escherichia coli* cells, *Streptomyces* cells, *Pichia pastoris* cells, *Schizosaccharomyces* cells, especially *Schizosaccharomyces pombe* cells, *Aspergillus* cells, and *Chlamydomonas* cells.

The plant cells and plants of the present invention may be in principle any plant cells or plants. Preferably, it is a monocotyledonous or dicotyledonous crop plant and more preferably a fodder plant. The most preferred plants are a *Nicotiana spec*., especially *Nicotiana benthamiana* and *Nicotiana tabacum, Pisum sativum, Hordeum vulgare, Zea mays, Cucumis sativus* and *Linum usitatissimum* and rapeseed. Other plants which may be used are e.g. flax, poppy, olive, cocoa, barley, wheat, almond, sesame, mustard, castor oil plant, sunflower, soybean, peanut, coconut, turnip seed, cotton, cucumber, tobacco and oil palm trees.

The use of peas (pisum sativum) showed surprising results. Self pollinating plants like pisum sativum are especially suited for the production of recombinant proteins.

Addionaly

The invention also relates to propagation material and agricultural products of the plants according to the invention, for example fruits, seeds, tubers, rootstocks, seedlings, cuttings and the like.

Especially the use of seeds is advantageous. The antibody constructs are protected from degradation due to protease inhibitors, which are present in most seeds. Therefore long-term storage does not influence the activity or the amount of protein.

It is another object of the invention to provide a composition comprising the antigen-binding polypeptide, the polynucleotide, the nucleic construct and/or the antibody construct of the present invention and an acceptable excipient, carrier, buffer and/or stabilizer.

Antigen-binding polypeptides in the state of art may be degraded rapidly after oral application. Therefore special attention has to be given to the high acidity in the stomach as well as the presence of secretory proteases, particularly in the oral cavity and in the small intestine. Therefore the excipient, carrier, buffer and/or stabilizer preferably comprises a protein source, such as BSA, milk powder and/or pea flour. The proteins may be added alone or as a protein mixture. Additionally or alternatively, the excipient, carrier, buffer and/or stabilizer may comprise methyl-cellulose.

Furthermore, the antigen binding peptides may be formulated as acid-resistant micro-particles, or be packed into acid-resistant capsules, preferably gelatine capsules. This embodiment is especially advantageous for oral administration.

Especially preferred is the composition, wherein the excipient, carrier, buffer and/or stabilizer comprises a protein source, preferably BSA, milk powder and/or pea flour; micro-particles and/or gelatine capsules.

The invention also relates to the use of the polypeptide, the antibody construct, the polynucleotide, the nucleic construct and/or the vector for the manufacture of a medicament for the treatment, diagnosis and/or prevention of infections, preferred infections associated with *Eimeria* species, especially *Eimeria tenella*.

In certain applications, the pharmaceutical compositions disclosed herein may be delivered via oral administration to an animal and/or a human. As such, these compositions may be formulated with an inert diluent or with an assimilable edible carrier, or they may be enclosed in hard- or soft-shell gelatin capsule, or they may be compressed into tablets, or they may be incorporated directly with the food of the diet.

The active compounds may even be incorporated with excipients and used in the form of ingestible tablets, buccal tables, troches, capsules, elixirs, suspensions, syrups, wafers, and the like. The tablets, troches, pills, capsules and the like may also contain the following: a binder, as gum tragacanth, acacia, cornstarch, or gelatin; excipients, such as dicalcium phosphate; a disintegrating agent, such as corn starch, potato starch, alginic acid and the like; a lubricant, such as magnesium stearate; and a sweetening agent, such as sucrose, lactose or Saccharin may be added or a flavoring agent, such as peppermint, oil of wintergreen, or cherry flavoring. When the dosage unit form is a capsule, it may contain, in addition to materials of the above type, a liquid carrier. Various other materials may be present as coatings or to otherwise modify the physical form of the dosage unit. For instance, tablets, pills, or capsules may be coated with shellac, sugar, or both. A syrup of elixir may contain the active compound sucrose as a sweetening agent methyl and propylparabens as preservatives, a dye and flavoring, such as cherry or orange flavor. Of course, any material used in preparing any dosage unit form should be pharmaceutically pure and substantially non-toxic in the amounts employed. In addition, the active compounds may be incorporated into sustained-release preparation and formulations.

In certain circumstances it will be desirable to deliver the pharmaceutical compositions disclosed herein parenterally, intravenously, intramuscularly, or even intraperitoneally. Solutions of the active compounds as free base or pharmacologically acceptable salts may be prepared in water suitably mixed with a surfactant, such as hydroxypropylcellulose. Dispersions may also be prepared in glycerol, liquid polyethylene glycols, and mixtures thereof and in oils. Under ordinary conditions of storage and use, these preparations contain a preservative to prevent the growth of microorganisms.

The pharmaceutical forms suitable for injectable use include sterile aqueous Solutions or dispersions and sterile powders for the extemporaneous preparation of sterile injectable Solutions or dispersions. In all cases the form must be sterile and must be fluid to the extent that easy syringability exists. It must be stable under the conditions of manufacture and storage and must be preserved against the contaminating action of microorganisms, such as bacteria and fungi. The carrier can be a solvent or dispersion medium containing, for example, water, ethanol, polyol (e.g., glycerol, propylene glycol, and liquid polyethylene glycol, and the like), suitable mixtures thereof, and/or vegetable oils. Proper fluidity may be maintained, for example, by the use of a coating, such as lecithin, by the maintenance of the required particle size in the case of dispersion and by the use of surfactants. The prevention of the action of microorganisms can be facilitated by various antibacterial and antifungal agents, for example, parabens, chlorobutanol, phenol, sorbic acid, thimerosal, and the like. In many cases, it will be preferable to include isotonic agents, for example, sugars or sodium Chloride.

Prolonged absorption of the injectable compositions can be brought about by the use in the compositions of agents delaying absorption, for example, aluminum monostearate and gelatin. For parenteral administration in an aqueous solution, for example, the solution should be suitably buffered if necessary and the liquid diluent first rendered isotonic with sufficient saline or glucose. These particular aqueous Solutions are especially suitable for intravenous, intramuscular, subcutaneous and intraperitoneal administration. In this connection, a sterile aqueous medium that can be employed will be known to those of skill in the art in light of the present disclosure. For example, one dosage may be dissolved in 1 ml of isotonic NaCI solution and either added to 1000 ml of hypodermoclysis fluid or injected at the proposed site of infusion. Some Variation in dosage will necessarily occur depending on the condition of the subject being treated. The person responsible for administration will, in any event, determine the appropriate dose for the individual subject. Moreover, for human administration, preparations should meet sterility, pyrogenicity, and the general safety and purity standards as required by national or regional Offices of biologics Standards.

Sterile injectable solutions are prepared by incorporating the active compounds in the required amount in the appropriate solvent with several of the other ingredients enumerated above, as required, followed by filtered sterilization. Generally, dispersions are prepared by incorporating the various sterilized active ingredients into a sterile vehicle which contains the basic dispersion medium and the required other ingredients from those enumerated above. In the case of sterile powders for the preparation of sterile injectable Solutions, the preferred methods of preparation are vacuum-drying and freeze-drying techniques which yield a powder of the active ingredient plus any additional desired ingredient from a previously sterile-filtered solution thereof.

The compositions disclosed herein may be formulated in a neutral or salt form. Pharmaceutically-acceptable salts include the acid addition salts (formed with the free amino groups of the protein) and which are formed with inorganic acids such as, for example, hydrochloric or phosphoric acids, or such organic acids as acetic, oxalic, tartaric, mandelic, and the like. Salts formed with the free carboxyl groups can also be derived from inorganic bases such as, for example, sodium, potassium, ammonium, calcium, or ferric hydroxides, and such organic bases as isopropylamine, trimethylamine, histidine, procaine and the like. Upon formulation, Solutions will be administered in a manner compatible with the dosage formulation and in such amount as is therapeutically effective. The formulations are easily administered in injectable Solutions, drug-release capsules, and the like.

As used herein, "carrier" includes any and all solvents, dispersion media, vehicles, coatings, diluents, antibacterial and antifingal agents, isotonic and absorption delaying agents, buffers, carrier Solutions, suspensions, colloids, and the like. The use of such media and agents for pharmaceutical active substances is well known in the art. Except insofar as any conventional media or agent is incompatible with the active ingredient, its use in the therapeutic compositions is contemplated. Supplementary active ingredients can also be incorporated into the compositions.

The phrase "pharmaceutically-acceptable" refers to molecular entities and compositions that do not produce an allergic or similar untoward reaction when administered to a human. The preparation of an aqueous composition that contains a protein as an active ingredient is well understood in the art. Typically, such compositions are prepared as injectables, either as liquid Solutions or suspensions; solid forms suitable for solution in, or Suspension in, liquid prior to injection can also be prepared. The preparation can also be emulsified.

Also preferred is the use of the plants, plant parts and/or microorganisms as fodder, preferred for production animals, more preferred birds, more preferred poultry, especially preferred chicken. Oral administration of antibodies specific to host pathogens is an attractive approach to establish protective immunity, especially against gastrointestinal pathogens.

It is understood that the oral administration is not limited to production animals but can also be applied to humans.

This embodiment simplifies the administration in an immense way. Due to the invention production animals can be treated without the need of a special administration step. Surprisingly the fodder can be used in prophylaxis as well.

One advantage of the invention is the fact that there is no toxic dose for the polypeptides of the invention. Therefore the administration via fodder is possible. This method saves a lot of time and costs, because no extra steps are necessary and a surprisingly high rate of yield was achieved.

Another advantage of this method is the fact that no isolation or purification of the antigen-binding peptide or antibody construct is necessary. In antibody production the isolation and purification is a time-consuming step and therefore expensive.

It was not expected, that the antigen-binding polypeptides or the antibody constructs of the invention show similar results when they are not isolated but administered in plants, parts thereof or microorganisms. Therefore it was surprising that the animals treated with fodder instead of purified antibodies showed comparable results.

The teachings of the present invention are characterised by the following features:
- departure from the beaten track
- a new perception of the problem
- satisfaction of a long-felt need or want
- hitherto all efforts of experts were in vain
- the simplicity of the solution, which proves inventive action, especially since it replaces a more complex doctrine
- the development of scientific technology followed another direction
- the achievement forwards the development
- misconceptions among experts about the solution of the according problem (prejudice)
- technical progress, such as: improvement, increased performance, price-reduction, saving of time, material, work steps, costs or resources that are difficult to obtain, improved reliability, remedy of defects, improved quality, no maintenance, increased efficiency, better yield, augmentation of technical possibilities, provision of another product, opening of a second way, opening of a new field, first solution for a task, spare product, alternatives, possibility of rationalisation, automation or miniaturisation or enrichment of the pharmaceutical fund
- special choice; since a certain possibility, the result of which was unforeseeable, was chosen among a great number of possibilities, it is a patentable lucky choice
- error in citations
- young field of technology
- combined invention; a combination of a number of known elements, with a surprising effect
- licensing
- praise of experts and
- commercial success

Said advantages are shown especially in the preferential embodiments of the invention.

### Examples

### Example 1:

### Analyses of antigen binding

### Antigen: oocyst extract

| Antibody | scFv AB28 | Di-kappa-body |
|---|---|---|
| K_{D} [nM] | 6.86 ± 1.56 | 0.55 ± 0.14 |

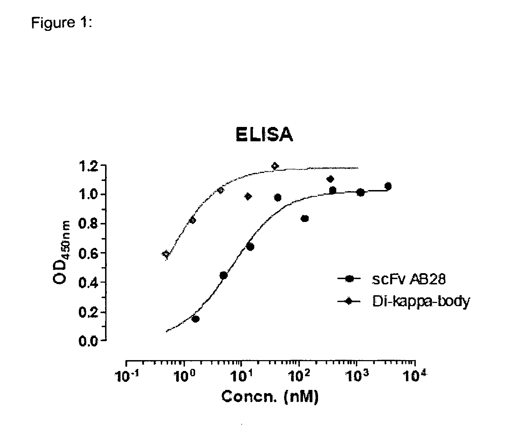

The results of this experiment, shown in figure 1, prove that Di-kappa-bodies are bivalent.

### Example 2:

### Comparison of chicken and piglet chymes

### Method:

Purified antibody fragments were incubated at 37°C for 30 min with serial dilutions of piglet or chicken chyme. Antigen-binding activity was tested by ELISA.

| **Ab fragment** | **Chyme*** | **ED₅₀** | **R²** |
|---|---|---|---|
| Di-kappa-body (tobacco) | piglet | 422.2 | 0.98 |
| | chicken | 458.3 | 0.97 |

| | | | |
|---|---|---|---|
| *Origin of used intestinal chyme ED50: effective dilution of chime leading to 50% loss of activity R2: goodness of fit | | | |

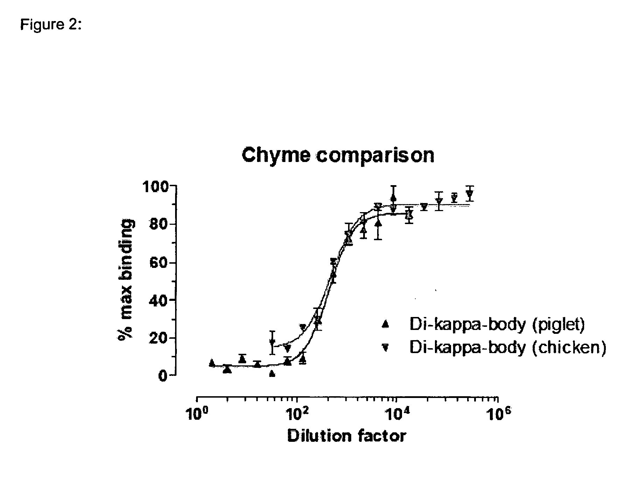

Results are shown in figure 2.

### Example 3:

**Table 1: Individual amino acid sequences of VH CDR1, VH CDR2 and VH CDR3 of the Eimeria-binding polypeptides AA19, AA28, AB09, AB21, AB28 and AD10.**

| | **VH CDR1** | **SEQ ID #** | **VH CDR2** | **SEQ ID #** | **VH CDR3** | **SEQ ID #** |
|---|---|---|---|---|---|---|
| AA28 | DHAIH | 11 | YFSPGNNDIKYNEKFKG | 17 | WPGAMDY | 23 |
| AB28 | VYIIH | 12 | YINPYNDGTKYNEKFRG | 18 | FYYGSSSVFDY | 24 |
| AD10 | SYAMS | 13 | SISSGGSTYYPDSVKG | 19 | NYGSYWGDSYFFDY | 25 |
| AB09 | NFWMH | 14 | EINPSNGDTNYNEKFKR | 20 | RGWLHFWYFDV | 26 |
| AB21 | NFWIH | 15 | EINPSNGDTKYNEKFRR | 21 | RGWLHFWYFDV | 26 |
| AA19 | DTYIH | 16 | RIDPANGNTKYDPKFQG | 22 | SLGDYFAMNY | 27 |

**Table 2: Individual amino acid sequences of VL CDR1, VL CDR2 and VL CDR3 of the Eimeria-binding polypeptides AA19, AA28, AB09, AB21, AB28 and AD10.**

| | VL CDR1 | SEQ ID # | VL CDR2 | SEQ ID # | VL CDR3 | SEQ ID # |
|---|---|---|---|---|---|---|
| AA28 | KASQDVGTAVA | 28 | WASTRHT | 32 | QQYSSYPLT | 36 |
| AB28 | KASQNVGTNVA | 29 | SASYRYS | 33 | QQYNSYPLT | 37 |
| AD10 | KASQNVGTNVA | 29 | SASYRYS | 33 | QQYNSYPLT | 37 |
| AB09 | KASQDVGTAVA | 28 | WASTRHT | 32 | HQYSSYPLT | 38 |
| AB21 | RSSQSLENSNGN TYLN | 30 | KVSNRFS | 34 | FQGSHVPYT | 39 |
| AA19 | RASGNIHNYLA | 31 | NAKTLAD | 35 | QHFWSTPPT | 40 |

## Claims

1. An antibody construct, comprising two polypeptide monomers, each polypeptide monomer comprising
- one constant light domain, wherein
the constant domains of both monomers are operatively connected, preferred via a disulfide bond,
- one variable light domain, wherein
the variable light domain is associated with the constant domain,
- one variable heavy domain, wherein
the variable heavy domain is connected to the variable light domain via a linker, wherein
said linker consists of 0-12 amino acids, whereby
the variable light domain of each monomer is operatively associated with the variable heavy domain of the other monomer, and
the variable light domain of each monomer forms an antigen binding site with the variable heavy domain of the other monomer.

2. An antibody construct according to any of the preceding claims, wherein
both constant domains of a light chain are kappa domains.

3. An antibody construct according to any of the preceding claims, wherein
both constant domains of a light chain are lambda domains.

4. An antibody construct according to any of the preceding claims, wherein
the constant domains are connected, preferred via a disulfide bridge and/or hydrophobic interaction.

5. An antibody construct according to any of the preceding claims, wherein
said polypeptide and/or an antibody construct is monospecific.

6. An antibody construct according to any of the preceding claims, wherein
said polypeptide and/or an antibody construct is bispecific.

7. An antibody construct according to any of the preceding claims, wherein
said linker is selected from the group consisting of a GS-linker, a yol-linker and/or a linker with the amino acid sequence comprising Seq. ID NO 41.

8. An antibody construct according to any of the preceding claims, wherein
at least one constant domain of a light chain C_{L} comprises the amino acid sequence of Seq. ID NO 10.

9. Antibody construct of any one of claims 1 to 8 for use as a medicament.

10. An antibody construct of any one of claims 1 to 8 for in vitro diagnosis.

11. A polynucleotide comprising a nucleotide sequence which encodes an antibody construct of any one of claims 1-8.

12. A host cell comprising the polynucleotide of claim 11.

13. A transgenic plant, parts thereof and/or microorganisms, containing the polynucleotide of claim 11.

14. A composition comprising the polynucleotide of claim 11 and/or an antibody construct of any one of claims 1 to 8 and a pharmaceutically acceptable excipient, carrier, buffer and/or stabilizer.

15. Plants, plant parts and/or microorganisms of claim 13 suitable for oral administration as fodder, preferred for birds, more preferred poultry, especially preferred chicken.

## Patentansprüche

1. Fusionsantikörper, der zwei Polypeptidmonomere umfasst, wobei jedes der Polypeptidmonomere Folgendes umfasst
- eine konstante leichte Domäne, wobei
die konstanten Domänen beider Monomere wirksam miteinander verknüpft sind, bevorzugt über eine Disulfidbindung,
- eine variable leichte Domäne, wobei
die variable leichte Domäne mit der konstanten Domäne in Verbindung steht,
- eine variable schwere Domäne, wobei
die variable schwere Domäne über einen Linker mit der variablen leichten Domäne verknüpft ist,
wobei
der Linker aus 0 bis 12 Aminosäuren besteht, wobei
die variable leichte Domäne jedes der Monomere mit der variablen schweren Domäne des jeweils anderen Monomers wirksam in Verbindung steht, und
die variable leichte Domäne jedes der Monomere mit der variablen schweren Domäne des jeweils anderen Monomers eine Antigen-Bindungsstelle bildet.

2. Fusionsantikörper gemäß einem beliebigen der vorhergehenden Ansprüche, wobei
es sich bei den beiden konstanten Domänen einer leichten Kette um kappa-Domänen handelt.

3. Fusionsantikörper gemäß einem beliebigen der vorhergehenden Ansprüche, wobei
es sich bei den beiden konstanten Domänen einer leichten Kette um lambda-Domänen handelt.

4. Fusionsantikörper gemäß einem beliebigen der vorhergehenden Ansprüche, wobei
die konstanten Domänen miteinander verknüpft sind, bevorzugt über eine Disulfidbrücke und/oder eine hydrophobe Wechselwirkung.

5. Fusionsantikörper gemäß einem beliebigen der vorhergehenden Ansprüche, wobei
das Polypeptid und/oder ein Fusionsantikörper monospezifisch ist.

6. Fusionsantikörper gemäß einem beliebigen der vorhergehenden Ansprüche, wobei
das Polypeptid und/oder ein Fusionsantikörper bispezifisch ist.

7. Fusionsantikörper gemäß einem beliebigen der vorhergehenden Ansprüche, wobei
der Linker aus der Gruppe ausgewählt ist, die aus einem GS-Linker, einem yol-Linker und/oder einem Linker besteht, dessen Aminosäuresequenz die Seq.-ID Nr. 41 umfasst.

8. Fusionsantikörper gemäß einem beliebigen der vorhergehenden Ansprüche, wobei
mindestens eine konstante Domäne einer leichten Kette C_{L} die Aminosäuresequenz der Seq.-Nr. 10 umfasst.

9. Fusionsantikörper nach einem beliebigen der Ansprüche 1 bis 8, zur Verwendung als Arzneimittel.

10. Fusionsantikörper nach einem beliebigen der Ansprüche 1 bis 8, zur invitro-Diagnose.

11. Polynukleotid, das eine Nukleotidsequenz umfasst, die für einen Fusionsantikörper nach einem beliebigen der Ansprüche 1 bis 8 codiert.

12. Wirtszelle, die das Polynukleotid nach Anspruch 11 umfasst.

13. Transgene Pflanze, Teile derselben und/oder Mikroorganismen, die das Polynukleotid nach Anspruch 11 enthalten.

14. Zusammensetzung, die das Polynukleotid nach Anspruch 11 und/oder einen Fusionsantikörper nach einem beliebigen der Ansprüche 1 bis 8 sowie einen pharmazeutisch unbedenklichen Hilfsstoff, Trägerstoff, Puffer und/oder Stabilisator umfasst.

15. Pflanzen, Pflanzenteile und/oder Mikroorganismen nach Anspruch 13, die sich zur oralen Verabreichung als Futtermittel eignen, bevorzugt für Vögel, insbesondere Nutzgeflügel, mit besonderem Vorzug Hühner.

## Revendications

1. Construction d'anticorps, comprenant deux monomères polypeptidiques, chaque monomère polypeptidique comprenant
- un domaine léger constant, dans lequel
les domaines constants des deux monomères sont liés opérationnellement, de préférence via une liaison disulfure,
- un domaine léger variable, dans lequel
le domaine léger variable est associé avec le domaine constant,
- un domaine lourd variable, dans lequel
le domaine lourd variable est lié au domaine léger variable via un lieur, dans lequel
ledit lieur est constitué de 0 à 12 acides aminés, moyennant quoi le domaine léger variable de chaque monomère est associé opérationnellement au domaine lourd variable de l'autre monomère, et
le domaine léger variable de chaque monomère forme un site de liaison d'antigène avec le domaine lourd variable de l'autre monomère.

2. Construction d'anticorps selon l'une quelconque des revendications précédentes, dans laquelle les deux domaines constants d'une chaîne légère sont des domaines kappa.

3. Construction d'anticorps selon l'une quelconque des revendications précédentes, dans laquelle les deux domaines constants d'une chaîne légère sont des domaines lambda.

4. Construction d'anticorps selon l'une quelconque des revendications précédentes, dans laquelle les domaines constants sont liés, de préférence via un pont disulfure et/ou une interaction hydrophobe.

5. Construction d'anticorps selon l'une quelconque des revendications précédentes, dans laquelle ledit polypeptide et/ou la construction d'anticorps est monospécifique.

6. Construction d'anticorps selon l'une quelconque des revendications précédentes, dans laquelle ledit polypeptide et/ou la construction d'anticorps est bispécifique.

7. Construction d'anticorps selon l'une quelconque des revendications précédentes, dans laquelle ledit lieur est choisi dans le groupe constitué par un GS-lieur, un yol-lieur et/ou un lieur avec la séquence d'acides aminés comprenant SEQ ID n° 41.

8. Construction d'anticorps selon l'une quelconque des revendications précédentes, dans laquelle au moins un domaine constant d'une chaîne légère C_{L}, comprend la séquence d'acides aminés de SEQ ID n° 10.

9. Anticorps selon l'une quelconque des revendications 1 à 8 pour une utilisation comme médicament.

10. Anticorps selon l'une quelconque des revendications 1 à 8 pour un diagnostic in vitro.

11. Polynucléotide comprenant une séquence nucléotidique qui code une construction d'anticorps selon l'une quelconque des revendications 1 à 8.

12. Cellule hôte comprenant le polynucléotide selon la revendication 11.

13. Plante transgénique, les parties associées et/ou les microorganismes, contenant le polynucléotide selon la revendication 11.

14. Composition comprenant le polynucléotide selon la revendication 11 et/ou une construction d'anticorps selon l'une quelconque des revendications 1 à 8 et un excipient, véhicule, tampon et/ou stabilisant pharmaceutiquement acceptable.

15. Plantes, parties de plantes et/ou microorganismes selon la revendication 13 adaptés pour une administration orale comme aliment, de préférence pour les oiseaux, davantage de préférence pour la volaille, particulièrement de préférence pour le poulet.


### SEQUENCE LISTING

<110> Novoplant GmbH
<120> Proteolytically stable antibody formats
<130> N.N.
<150> 10 2008 050 749.0
   <151> 2008-10-06
<150> 10 2008 050 757.1
   <151> 2008-10-07
<160> 41
<170> PatentIn version 3.3
<210> 1
   <211> 340
   <212> PRT
   <213> Human
<220>
   <221> MISC_FEATURE
   <222> (340)..(340)
   <223> /note = optional
<400> 1
   Gln Val Gln Leu Gln Gln Ser Gly Pro Glu Leu Val Lys Pro Gly Ala
   1               5                   10                  15
   Ser Val Lys Met Ser Cys Lys Ala Ser Gly Tyr Thr Phe Thr Val Tyr
               20                  25                  30
   Ile Ile His Trp Leu Lys Gln Lys Pro Gly Gln Gly Leu Glu Trp Ile
           35                  40                  45
   Gly Tyr Ile Asn Pro Tyr Asn Asp Gly Thr Lys Tyr Asn Glu Lys Phe
       50                  55                  60
   Arg Gly Lys Ala Thr Leu Thr Ser Asp Lys Ser Ser Ser Thr Ala Tyr
   65                  70                  75                  80
   Met Glu Leu Ser Ser Leu Thr Ser Glu Asp Ser Ala Val Tyr Tyr Cys
                   85                  90                  95
   Ala Arg Phe Tyr Tyr Gly Ser Ser Ser Val Phe Asp Tyr Trp Gly Gln
               100                 105                 110
   Gly Thr Thr Leu Thr Val Ser Ser Gly Gly Ser Gly Gly Ser Asp Val
           115                 120                 125
   Val Met Thr Gln Thr Gln Lys Phe Met Ser Thr Ser Val Gly Asp Arg
       130                 135                 140
   Val Ser Val Thr Cys Lys Ala Ser Gln Asn Val Gly Thr Asn Val Ala
   145 150 155 160
   Trp Tyr Gln Gln Lys Pro Gly Gln Ser Pro Lys Ala Leu Ile Tyr Ser
   165 170 175
   Ala Ser Tyr Arg Tyr Ser Gly Val Pro Asp Arg Phe Thr Gly Ser Gly
   180 185 190
   Ser Gly Thr Asp Phe Thr Leu Thr Ile Ser Asn Val Gln Ser Glu Asp
   195 200 205
   Leu Ala Glu Tyr Phe Cys Gln Gln Tyr Asn Ser Tyr Pro Leu Thr Phe
   210 215 220
   Gly Ala Gly Thr Lys Leu Glu Leu Lys Arg Thr Val Ala Ala Pro Ser
   225 230 235 240
   Val Phe Ile Phe Pro Pro Ser Asp Glu Gln Leu Lys Ser Gly Thr Ala
   245 250 255
   Ser Val Val Cys Leu Leu Asn Asn Phe Tyr Pro Arg Glu Ala Lys Val
   260 265 270
   Gln Trp Lys Val Asp Asn Ala Leu Gln Ser Gly Asn Ser Gln Glu Ser
   275 280 285
   Val Thr Glu Gln Asp Ser Lys Asp Ser Thr Tyr Ser Leu Ser Ser Thr
   290 295 300
   Leu Thr Leu Ser Lys Ala Asp Tyr Glu Lys His Lys Val Tyr Ala Cys
   305 310 315 320
   Glu Val Thr His Gln Gly Leu Ser Ser Pro Val Thr Lys Ser Phe Asn
   325 330 335
   Arg Gly Glu Cys
   340
<210> 2
   <211> 343
   <212> PRT
   <213> Human
<400> 2
   Glu Val Gln Leu Val Glu Ser Gly Gly Gly Leu Val Lys Ala Gly Gly
   1               5                   10                  15
   Ser Leu Ile Leu Ser Cys Gly Val Ser Asn Phe Arg Ile Ser Ala His
               20                  25                  30
   Thr Met Asn Trp Val Arg Arg Val Pro Gly Gly Gly Leu Glu Trp Val
           35                  40                  45
   Ala Ser Ile Ser Thr Ser Ser Thr Tyr Arg Asp Tyr Ala Asp Ala Val
   50 55 60
   Lys Gly Arg Phe Thr Val Ser Arg Asp Asp Leu Glu Asp Phe Val Tyr
   65 70 75 80
   Leu Gln Met His Lys Met Arg Val Glu Asp Thr Ala Ile Tyr Tyr Cys
   85 90 95
   Ala Arg Lys Gly Ser Asp Arg Leu Ser Asp Asn Asp Pro Phe Asp Ala
   100 105 110
   Trp Gly Pro Gly Thr Val Val Thr Val Ser Pro Gly Gly Ser Gly Gly
   115 120 125
   Ser Asp Val Val Met Thr Gln Ser Pro Ser Thr Leu Ser Ala Ser Val
   130 135 140
   Gly Asp Thr Ile Thr Ile Thr Cys Arg Ala Ser Gln Ser Ile Glu Thr
   145 150 155 160
   Trp Leu Ala Trp Tyr Gln Gln Lys Pro Gly Lys Ala Pro Lys Leu Leu
   165 170 175
   Ile Tyr Lys Ala Ser Thr Leu Lys Thr Gly Val Pro Ser Arg Phe Ser
   180 185 190
   Gly Ser Gly Ser Gly Thr Glu Phe Thr Leu Thr Ile Ser Gly Leu Gln
   195 200 205
   Phe Asp Asp Phe Ala Thr Tyr His Cys Gln His Tyr Ala Gly Tyr Ser
   210 215 220
   Ala Thr Phe Gly Gln Gly Thr Arg Val Glu Ile Lys Arg Thr Val Ala
   225 230 235 240
   Ala Pro Ser Val Phe Ile Phe Pro Pro Ser Asp Glu Gln Leu Lys Ser
   245 250 255
   Gly Thr Ala Ser Val Val Cys Leu Leu Asn Asn Phe Tyr Pro Arg Glu
   260 265 270
   Ala Lys Val Gln Trp Lys Val Asp Asn Ala Leu Gln Ser Gly Asn Ser
   275 280 285
   Gln Glu Ser Val Thr Glu Gln Asp Ser Lys Asp Ser Thr Tyr Ser Leu
   290 295 300
   Ser Ser Thr Leu Thr Leu Ser Lys Ala Asp Tyr Glu Lys His Lys Val
   305 310 315 320
   Tyr Ala Cys Glu Val Thr His Gln Gly Leu Ser Ser Pro Val Thr Lys
                   325                 330                 335
   Ser Phe Asn Arg Gly Glu Cys
               340
<210> 3
   <211> 372
   <212> PRT
   <213> Human
<400> 3
<210> 4
   <211> 120
   <212> PRT
   <213> Mouse
<400> 4
   Gln Val Gln Leu Gln Gln Ser Gly Pro Glu Leu Val Lys Pro Gly Ala
   1 5 10 15
   Ser Val Lys Met Ser Cys Lys Ala Ser Gly Tyr Thr Phe Thr Val Tyr
   20 25 30
   Ile Ile His Trp Leu Lys Gln Lys Pro Gly Gln Gly Leu Glu Trp Ile
   35 40 45
   Gly Tyr Ile Asn Pro Tyr Asn Asp Gly Thr Lys Tyr Asn Glu Lys Phe
   50 55 60
   Arg Gly Lys Ala Thr Leu Thr Ser Asp Lys Ser Ser Ser Thr Ala Tyr
   65 70 75 80
   Met Glu Leu Ser Ser Leu Thr Ser Glu Asp Ser Ala Val Tyr Tyr Cys
   85 90 95
   Ala Arg Phe Tyr Tyr Gly Ser Ser Ser Val Phe Asp Tyr Trp Gly Gln
   100 105 110
   Gly Thr Thr Leu Thr Val Ser Ser
   115 120
<210> 5
   <211> 123
   <212> PRT
   <213> Human
<400> 5
   Glu Val Gln Leu Val Glu Ser Gly Gly Gly Leu Val Lys Ala Gly Gly
   1 5 10 15
   Ser Leu Ile Leu Ser Cys Gly Val Ser Asn Phe Arg Ile Ser Ala His
   20 25 30
   Thr Met Asn Trp Val Arg Arg Val Pro Gly Gly Gly Leu Glu Trp Val
   35 40 45
   Ala Ser Ile Ser Thr Ser Ser Thr Tyr Arg Asp Tyr Ala Asp Ala Val
   50 55 60
   Lys Gly Arg Phe Thr Val Ser Arg Asp Asp Leu Glu Asp Phe Val Tyr
   65 70 75 80
   Leu Gln Met His Lys Met Arg Val Glu Asp Thr Ala Ile Tyr Tyr Cys
   85 90 95
   Ala Arg Lys Gly Ser Asp Arg Leu Ser Asp Asn Asp Pro Phe Asp Ala
   100 105 110
   Trp Gly Pro Gly Thr Val Val Thr Val Ser Pro
   115 120
<210> 6
   <211> 131
   <212> PRT
   <213> Human
<400> 6
   Thr Leu Lys Glu Ser Gly Pro Pro Leu Val Lys Pro Thr Gln Thr Leu
   1 5 10 15
   Thr Leu Thr Cys Ser Phe Ser Gly Phe Ser Leu Ser Asp Phe Gly Val
   20 25 30
   Gly Val Gly Trp Ile Arg Gln Pro Pro Gly Lys Ala Leu Glu Trp Leu
   35 40 45
   Ala Ile Ile Tyr Ser Asp Asp Asp Lys Arg Tyr Ser Pro Ser Leu Asn
   50 55 60
   Thr Arg Leu Thr Ile Thr Lys Asp Thr Ser Lys Asn Gln Val Val Leu
   65 70 75 80
   Val Met Thr Arg Val Ser Pro Val Asp Thr Ala Thr Tyr Phe Cys Ala
   85 90 95
   His Arg Arg Gly Pro Thr Thr Leu Phe Gly Val Pro Ile Ala Arg Gly
   100 105 110
   Pro Val Asn Ala Met Asp Val Trp Gly Gln Gly Ile Thr Val Thr Ile
   115 120 125
   Ser Ser Thr
   130
<210> 7
   <211> 107
   <212> PRT
   <213> Mouse
<400> 7
   Asp Val Val Met Thr Gln Thr Gln Lys Phe Met Ser Thr Ser Val Gly
   1 5 10 15
   Asp Arg Val Ser Val Thr Cys Lys Ala Ser Gln Asn Val Gly Thr Asn
   20 25 30
   Val Ala Trp Tyr Gln Gln Lys Pro Gly Gln Ser Pro Lys Ala Leu Ile
   35 40 45
   Tyr Ser Ala Ser Tyr Arg Tyr Ser Gly Val Pro Asp Arg Phe Thr Gly
   50 55 60
   Ser Gly Ser Gly Thr Asp Phe Thr Leu Thr Ile Ser Asn Val Gln Ser
   65 70 75 80
   Glu Asp Leu Ala Glu Tyr Phe Cys Gln Gln Tyr Asn Ser Tyr Pro Leu
   85 90 95
   Thr Phe Gly Ala Gly Thr Lys Leu Glu Leu Lys
   100 105
<210> 8
   <211> 107
   <212> PRT
   <213> Human
<400> 8
   Asp Val Val Met Thr Gln Ser Pro Ser Thr Leu Ser Ala Ser Val Gly
   1 5 10 15
   Asp Thr Ile Thr Ile Thr Cys Arg Ala Ser Gln Ser Ile Glu Thr Trp
   20 25 30
   Leu Ala Trp Tyr Gln Gln Lys Pro Gly Lys Ala Pro Lys Leu Leu Ile
   35 40 45
   Tyr Lys Ala Ser Thr Leu Lys Thr Gly Val Pro Ser Arg Phe Ser Gly
   50 55 60
   Ser Gly Ser Gly Thr Glu Phe Thr Leu Thr Ile Ser Gly Leu Gln Phe
   65 70 75 80
   Asp Asp Phe Ala Thr Tyr His Cys Gln His Tyr Ala Gly Tyr Ser Ala
   85 90 95
   Thr Phe Gly Gln Gly Thr Arg Val Glu Ile Lys
   100 105
<210> 9
   <211> 128
   <212> PRT
   <213> Human
<400> 9
   Asp Met Arg Val Pro Ala Gln Leu Leu Gly Leu Leu Leu Leu Trp Leu
   1               5                   10                  15
   Pro Gly Ala Arg Cys Ala Leu Gln Leu Thr Gln Ser Pro Ser Ser Leu
               20                  25                  30
   Ser Ala Ser Val Gly Asp Arg Ile Thr Ile Thr Cys Arg Ala Ser Gln
           35                  40                  45
   Gly Val Thr Ser Ala Leu Ala Trp Tyr Arg Gln Lys Pro Gly Ser Pro
       50                  55                  60
   Pro Gln Leu Leu Ile Tyr Asp Ala Ser Ser Leu Glu Ser Gly Val Pro
   65                  70                  75                  80
   Ser Arg Phe Ser Gly Ser Gly Ser Gly Thr Glu Phe Thr Leu Thr Ile
                   85                  90                  95
   Ser Thr Leu Arg Pro Glu Asp Phe Ala Thr Tyr Tyr Cys Gln Gln Leu
               100                 105                 110
   His Phe Tyr Pro His Thr Phe Gly Gly Gly Thr Arg Val Asp Val Arg
               115                 120                 125
<210> 10
   <211> 107
   <212> PRT
   <213> Human
<400> 10
<210> 11
   <211> 5
   <212> PRT
   <213> Mouse
<400> 11
   Asp His Ala Ile His
   1 5
<210> 12
   <211> 5
   <212> PRT
   <213> Mouse
<400> 12
   Val Tyr Ile Ile His
   1 5
<210> 13
   <211> 5
   <212> PRT
   <213> Mouse
<400> 13
   Ser Tyr Ala Met Ser
   1 5
<210> 14
   <211> 5
   <212> PRT
   <213> Mouse
<400> 14
   Asn Phe Trp Met His
   1 5
<210> 15
   <211> 5
   <212> PRT
   <213> Mouse
<400> 15
   Asn Phe Trp Ile His
   1 5
<210> 16
   <211> 5
   <212> PRT
   <213> Mouse
<400> 16
   Asp Thr Tyr Ile His
   1 5
<210> 17
   <211> 17
   <212> PRT
   <213> Mouse
<400> 17
   Tyr Phe Ser Pro Gly Asn Asn Asp Ile Lys Tyr Asn Glu Lys Phe Lys
   1 5 10 15
   Gly
<210> 18
   <211> 17
   <212> PRT
   <213> Mouse
<400> 18
   Tyr Ile Asn Pro Tyr Asn Asp Gly Thr Lys Tyr Asn Glu Lys Phe Arg
   1 5 10 15
   Gly
<210> 19
   <211> 16
   <212> PRT
   <213> Mouse
<400> 19
   Ser Ile Ser Ser Gly Gly Ser Thr Tyr Tyr Pro Asp Ser Val Lys Gly
   1 5 10 15
<210> 20
   <211> 17
   <212> PRT
   <213> Mouse
<400> 20
   Glu Ile Asn Pro Ser Asn Gly Asp Thr Asn Tyr Asn Glu Lys Phe Lys
   1 5 10 15
   Arg
<210> 21
   <211> 17
   <212> PRT
   <213> Mouse
<400> 21
   Glu Ile Asn Pro Ser Asn Gly Asp Thr Lys Tyr Asn Glu Lys Phe Arg
   1 5 10 15
   Arg
<210> 22
   <211> 17
   <212> PRT
   <213> Mouse
<400> 22
   Arg Ile Asp Pro Ala Asn Gly Asn Thr Lys Tyr Asp Pro Lys Phe Gln
   1 5 10 15
   Gly
<210> 23
   <211> 7
   <212> PRT
   <213> Mouse
<400> 23
   Trp Pro Gly Ala Met Asp Tyr
   1 5
<210> 24
   <211> 11
   <212> PRT
   <213> Mouse
<400> 24
   Phe Tyr Tyr Gly Ser Ser Ser Val Phe Asp Tyr
   1 5 10
<210> 25
   <211> 14
   <212> PRT
   <213> Mouse
<400> 25
   Asn Tyr Gly Ser Tyr Trp Gly Asp Ser Tyr Phe Phe Asp Tyr
   1 5 10
<210> 26
   <211> 11
   <212> PRT
   <213> Mouse
<400> 26
   Arg Gly Trp Leu His Phe Trp Tyr Phe Asp Val
   1 5 10
<210> 27
   <211> 10
   <212> PRT
   <213> Mouse
<400> 27
   Ser Leu Gly Asp Tyr Phe Ala Met Asn Tyr
   1 5 10
<210> 28
   <211> 11
   <212> PRT
   <213> Mouse
<400> 28
   Lys Ala Ser Gln Asp Val Gly Thr Ala Val Ala
   1 5 10
<210> 29
   <211> 11
   <212> PRT
   <213> Mouse
<400> 29
   Lys Ala Ser Gln Asn Val Gly Thr Asn Val Ala
   1 5 10
<210> 30
   <211> 16
   <212> PRT
   <213> Mouse
<400> 30
   Arg Ser Ser Gln Ser Leu Glu Asn Ser Asn Gly Asn Thr Tyr Leu Asn
   1 5 10 15
<210> 31
   <211> 11
   <212> PRT
   <213> Mouse
<400> 31
   Arg Ala Ser Gly Asn Ile His Asn Tyr Leu Ala
   1 5 10
<210> 32
   <211> 7
   <212> PRT
   <213> Mouse
<400> 32
   Trp Ala Ser Thr Arg His Thr
   1 5
<210> 33
   <211> 7
   <212> PRT
   <213> Mouse
<400> 33
   Ser Ala Ser Tyr Arg Tyr Ser
   1 5
<210> 34
   <211> 7
   <212> PRT
   <213> Mouse
<400> 34
   Lys Val Ser Asn Arg Phe Ser
   1 5
<210> 35
   <211> 7
   <212> PRT
   <213> Mouse
<400> 35
   Asn Ala Lys Thr Leu Ala Asp
   1               5
<210> 36
   <211> 9
   <212> PRT
   <213> Mouse
<400> 36
   Gln Gln Tyr Ser Ser Tyr Pro Leu Thr
   1               5
<210> 37
   <211> 9
   <212> PRT
   <213> Mouse
<400> 37
   Gln Gln Tyr Asn Ser Tyr Pro Leu Thr
   1               5
<210> 38
   <211> 9
   <212> PRT
   <213> Mouse
<400> 38
   His Gln Tyr Ser Ser Tyr Pro Leu Thr
   1               5
<210> 39
   <211> 9
   <212> PRT
   <213> Mouse
<400> 39
   Phe Gln Gly Ser His Val Pro Tyr Thr
   1               5
<210> 40
   <211> 9
   <212> PRT
   <213> Mouse
<400> 40
   Gln His Phe Trp Ser Thr Pro Pro Thr
   1               5
<210> 41
   <211> 6
   <212> PRT
   <213> Mouse
<400> 41
   Gly Gly Ser Gly Gly Ser
   1               5